(19) Europäisches Patentamt
European Patent Office
Office européen des brevets

(11) **EP 4 041 189 B1**

(12) **EUROPEAN PATENT SPECIFICATION**

(45) Date of publication and mention
of the grant of the patent:
**17.09.2025 Bulletin 2025/38**

(21) Application number: **20797620.0**

(22) Date of filing: **07.10.2020**

(51) International Patent Classification (IPC):
***A61K 9/00*** (2006.01)

(52) Cooperative Patent Classification (CPC):
**A61K 9/006; A61K 9/0024; A61K 9/0092**

(86) International application number:
**PCT/US2020/054617**

(87) International publication number:
**WO 2021/071974 (15.04.2021 Gazette 2021/15)**

(54) **ORALLY IMPLANTABLE DRUG DELIVERY DEVICE**

ORAL IMPLANTIERBARE WIRKSTOFFFREISETZUNGSVORRICHTUNG

DISPOSITIF D'ADMINISTRATION DE MÉDICAMENT POUVANT ÊTRE IMPLANTÉ PAR VOIE ORALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.10.2019 US 201962911765 P**
**27.11.2019 US 201962941267 P**
**05.08.2020 US 202063061483 P**

(43) Date of publication of application:
**17.08.2022 Bulletin 2022/33**

(73) Proprietor: **Oak Crest Institute of Science Monrovia, CA 91016 (US)**

(72) Inventors:
• **BAUM, Marc, M.**
**Pasadena, CA 91107 (US)**
• **MOSS, John, A.**
**Sierra Madre, CA 91024 (US)**
• **ANTON, Peter, A.**
**Monrovia, CA 91016 (US)**

(74) Representative: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) References cited:
**WO-A1-2016/149561     US-A- 4 020 558**

## Description

### FIELD OF INVENTION

This disclosure generally relates to implantable sustained release drug delivery devices.

### CROSS-REFERENCE TO RELATED APPLICATIONS

Priority is claimed to each of U.S.S.N. 62/911,765, filed October 7, 2019; U.S.S.N. 62/941,267, filed November 27, 2019; and U.S.S.N. 63/061,483, filed August 5, 2020.

### BACKGROUND

The subject matter disclosed herein is generally in the field of implantable drug delivery devices, and more particularly in the field of devices for the controlled, sustained release of a drug from a device implantable in the buccal mucosa of the oral cavity.

Drug delivery is an important aspect of medicine. The efficacy of many drugs is directly related to their administration route. Various systemic methods of drug delivery include oral, intravenous, intramuscular, and transdermal. These systemic methods may produce undesirable side effects and may result in reduced drug bioavailability and/or the metabolization of the drug by physiological processes, ultimately reducing the quantity of drug to reach the target site.

Accordingly, a variety of devices and methods have been developed to deliver drug efficiently and in a controlled fashion to the target compartment, while minimizing undesirable side-effects. Sustained release, or long-acting, formulations are known in the art to improve drug pharmacokinetic profiles compared to pulsatile delivery modalities (e.g., pills and injections) by maintaining steady-state plasma concentrations and avoiding the characteristic concentration peaks and troughs. This can lead to a lower occurrence of side-effects and lower required drug doses. In addition, sustained release drug delivery can improve adherence to therapy.

Adherence to frequent dosing is burdensome to the user and is a key factor in determining the efficacy outcomes of many therapeutic and prophylactic regimens. It is weil established across different delivery methods that adherence to therapy is inversely related to dosing period. By reducing dosing frequency, sustained release drug formulations inherently have the potential of increasing the efficacy of the regimen.

Implantable drug delivery devices are known in the art to represent a viable platform for systemic dosing taking advantage of the benefits of sustained release administration of the agent. However, there are a number of concerns related to the ability of existing devices to control the delivery of the medicine over time, and the predictability of the associated release kinetics, thereby resulting in poor therapeutic benefit. WO2016/149561A1 discloses a subdermal implant for the sustained release of water-soluble drugs. Further prior art is US4020558A.

For example, the drug eluting stent is a popular drug delivery device. Stents are mesh-like steel or plastic tubes that are used to open up a clogged atherosclerotic coronary artery or a blood vessel undergoing stenosis. A drug may be attached onto, or impregnated into, the stent that is believed to prevent re-clogging or restenosis a blood vessel. However, the initial release of the drug may be very rapid, releasing 20-40% of the total drug in a single day. Such high concentrations of the drug have been reported to result in cytotoxicity at the targeted site.

Implant devices targeting systemic drug delivery known in the art commonly are placed subdermally using a trocar. For example, subdermal implants delivering progestin-only contraceptives have been well-known in the art since the mid-1970s, with a number of FDA-approved products available, and offer warnen highly effective options for contraception without the side effects or risks associated with taking estrogen ( 1-3). Limitations of this common modality include: the need for a medical care professional for insertion and removal, along with access to a clinic; possible migration of the devices during use, making them difficult to remove; infection associated with non-sterile implantation technique; elevated foreign body response to the implants resulting in fibrosis; inflammation, with possible concomitant exudate, often with resultant fibrosis; and tactile or visual evidence of the implant resulting in stigmatization.

As a result of these challenges, there is a need for an implantable drug delivery device for administering an active agent to a subject (e.g., systemic dosing) that can be optimized to deliver any therapeutic, diagnostic, or prophylactic agent for any time period up to several years maintaining a controlled and desired rate.

### SUMMARY

The present invention is defined by independent claim 1. Embodiments of the invention are the subject-matter of the dependent claims. Provided herein are buccal implant devices configured to provide sustained drug delivery, the buccal implant device comprising: a body having a non-cylindrical geometry, the body adapted to be disposed within the oral mucosa of a patient; and one or more active pharmaceutical ingredients disposed within the body.

Also provided are buccal implant devices configured to provide sustained drug delivery, the buccal implant device comprising: a body adapted to be disposed within the oral mucosa of a patient; means for guiding the body into or out of the oral cavity of a patient; and one or more active pharmaceutical ingredients disposed within the body.

Further provided is a method of treating or preventing a medical condition in a patient in need thereof, comprising implanting a sustained release drug delivery device into the oral mucosa of a subject, the device comprising a matrix, reservoir, or hybrid design comprising one or more thermoplastic polymers, elastomer materials, or metals suitable for pharmaceutical use and a therapeutically effective amount of one or more active pharmaceutical ingredients. In some cases, the sustained release drug delivery device is a buccal implant device disclosed herein. The disclosure further provides a method of providing sustained, long term release of an active agent to a subject using the materials and methods described herein.

## BRIEF DESCRIPTION OF THE DRAWINGS

**FIG 1(A) and 1(B).** (A) Schematic representation of the oral cavity anatomy; arrows indicate four possible buccal compartments for implant placement. 1, outer lip; 2, hard plate; 3, palatine tonsil; 4, ventral side of tongue; 5, floor of mouth; 6, gingivae; 7, buccal mucosa; 8, inner lip; 9, gingivae. (B) Schematic representation of the oral cavity anatomy. 10, throat opening; 11, hard palate; 12, second upper molar; 13, parotid duct opening; 14, buccal mucosa; 15, retromolar trigone; 16, anterior tonsillar pillar; 17, tonsil.

**FIG 2.** Exemplary embodiments of buccal implant geometries (100 - 103).

**FIG 3.** Exemplary embodiments of buccal implant geometries (104 - 107).

**FIG 4.** Exemplary embodiment of buccal implant geometry (108).

**FIG 5.** Exemplary embodiment of buccal implant geometry (109).

**FIG 6.** Exemplary embodiment of buccal implant geometry (110).

**FIG 7.** Exemplary embodiments of buccal implant geometries (111 - 112).

**FIG 8.** Exemplary embodiments of matrix-type buccal implant designs (200 - 203).

**FIG 9.** Exemplary embodiments of segmented matrix-type buccal implant designs (204 - 205).

**FIG 10.** Exemplary embodiments of reservoir-type buccal implant designs (206).

**FIG 11.** Exemplary embodiments of segmented reservoir-type buccal implant designs (207 - 209).

**FIG 12.** Exemplary embodiment of hybrid-type buccal implant design (300 - 302).

**FIG 13.** Exemplary embodiments of hybrid-type buccal implant designs (303 - 304).

**FIG 14.** Exemplary embodiments of hybrid-type buccal implant designs comprising a plurality of skins (305).

**FIG 15.** Exemplary embodiment of hybrid-type buccal implant design (400 - 403).

**FIG 16.** Exemplary embodiments of hybrid-type buccal implant designs (401 - 414).

**FIG 17.** Exemplary embodiment of hybrid-type buccal implant design (415 - 420).

**FIG 18.** Exemplary embodiments of hybrid-type buccal implant designs (500 - 510).

**FIG 19** shows target Density Specifications for the Custom-extruded ePTFE Tubes. Grey bars, predicted densities; error bars, predicted density tolerance; black filled circles, measured densities.

**FIG 20A** shows *In Vitro* Release Kinetics of Prototype ePTFE TAF Implants. Slopes of the linear regression of the release data are used to calculate daily release rates (best fit values $\pm$ *SE*): 0.34 g cm$^{-3}$, 1.22 $\pm$ 0.023 mg d$^{-1}$ ($R^2 = 0.9921$); 0.84 g cm$^{-3}$, 0.58 $\pm$ 0.0089 mg d$^{-1}$ ($R^2 = 0.9941$); 0.47 g cm$^{-3}$, 0.40 $\pm$ 0.0087 mg d$^{-1}$ ($R^2 = 0.9895$); 1.13 g cm$^{-3}$, 0.12 $\pm$ 0.0037 mg d$^{-1}$ ($R^2 = 0.9798$). Densities correspond to the actual ePTFE tube density.

**FIG 20B** shows *In Vitro* Release Kinetics of Prototype ePTFE TAF Implants. Slopes of the linear regression of the release data are used to calculate daily release rates (best fit values $\pm$ *SE*) and are compared as a function of ePTFE density.

**FIG 21** shows the 90-day cumulative TAF release (median $\pm$ 95% *CI*) from 40 mm long, 2.4 mm outer dia. ePTFE (p = 0.84 g cm$^{-3}$) implants (*N* = 6) filled with a paste (141.8 $\pm$ 2.3 mg) consisting of TAF (70% w/w) blended with triethyl citrate (TEC).

**FIG 22A** and **22B** show the 80-day cumulative TAF release (median $\pm$ 95% *CI*) from 40 mm long, 2.4 mm outer dia. ePTFE ($\rho = 0.84$ g cm$^{-3}$) implants (*N* = 4) filled with a paste (140.8 $\pm$ 2.2 mg) consisting of TAF (77% w/w) blended with PEG 400. **FIG. 22A** uses the same y-axis range as **FIG. 21** for ease of comparison, while **FIG. 22B** shows the data with a zoomed y-axis.

## DETAILED DESCRIPTION

This disclosure is related to the use of a buccal implant as a device to deliver biologically active compounds at a controlled rate for an extended period of time, and methods of manufacture thereof. The device comprises at least one pharmaceutically active substance, and an unmedicated, pharmaceutically acceptable framework. The device is biocompatible and biostable, and is useful as an implant in patients (humans and animals), in need thereof, for the delivery of appropriate bioactive substances to tissues or organs.

Provided herein is a buccal implant device configured to provide sustained drug delivery, the buccal implant device comprising: a body having a non-cylindrical geometry, the body adapted to be disposed within the oral mucosa of a patient; and one or more active pharmaceutical ingredients disposed within the body. Also provided are buccal implant devices configured to provide sustained drug delivery, the buccal implant device comprising: a body adapted to be disposed within the oral mucosa of a patient; means for guiding the body into or out of the oral cavity of a patient; and one or more active pharmaceutical ingredients disposed within the body.

Further provided are methods of treating or preventing a medical condition in a patient in need thereof, comprising implanting a sustained release drug delivery device into the oral mucosa of a subject, the device comprising a matrix, reservoir, or hybrid design comprising one or more thermoplastic polymers, elastomer materials, or metals suitable for pharmaceutical use and a therapeutically effective amount of one or more active pharmaceutical ingredients.

Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (Boca Raton, FL, 2008); Oxford Textbook of Medicine, Oxford Univ. Press (Oxford, England, UK, May 2010, with 2018 update); Harrison's Principles of Internal Medicine, Vol .1 and 2, 20th ed., McGraw-Hill (New York, NY, 2018); Singleton and Sainsbury, Dictionary of Microbiology and Molecular Biology, 3rd ed., revised ed., J. Wiley & Sons (New York, NY, 2006); Smith, March's Advanced Organic Chemistry Reactions, Mechanisms and Structure 7th ed., J. Wiley & Sons (New York, NY, 2013); and Singleton, Dictionary of DNA and Genome Technology, 3rd ed., Wiley-Blackwell (Hoboken, NJ, 2012), provide one skilled in the art with a general guide to many of the terms used in the present application.

One skilled in the art will recognize many methods and materials similar or equivalent to those described herein, which could be used in the practice of the subject matter of the disclosure. Indeed, the present disclosure is in no way limited to the methods and materials described. For purposes of the present disclosure, certain terms are defined below.

"Conditions" and "disease conditions," include, but are not limited to, treating, preventing, reducing the likelihood of having, reducing the severity of, and/or slowing the progression of a medical condition in a subject, termed "application" hereunder. Such conditions or applications can be remedied through the use of one or more agents administered through a buccal sustained release agent delivery device.

As used herein, "treating" a medical condition includes reducing the severity of and/or slowing the progression of the medical condition. As used herein, "preventing" a medical condition includes, e.g., reducing the likelihood of having the medical condition or delaying the onset of a medical condition.

These conditions, or applications, are described further under "Use and Applications of the Device" and may include, but are in no way limited to, infectious diseases (e.g., a human immunodeficiency virus (HIV) infection, acquired immune deficiency syndrome (AIDS), a herpes simplex virus (HSV) infection, a hepatitis virus infection, respiratory viral infections (including but not limited to influenza viruses and coronaviruses, for example SARS-CoV-2)tuberculosis, other bacterial or viral infections, and malaria), diabetes, cardiovascular disorders, cancers, autoimmune diseases, central nervous system (CNS) conditions, and analogous conditions in non-human mammals.

In addition, the disclosure includes the administration of biologics, such as proteins and peptides, for the treatment or prevention of a variety of disorders such as conditions treatable with leuprolide (e.g., anemia caused by bleeding from uterine leiomyomas, fibroid tumors in the uterus, cancer of the prostate, and central precocious puberty), exenatide for the treatment of diabetes, histrelin acetate for the treatment for central precocious puberty, etc. A more detailed list of illustrative examples of potential applications of the disclosed disclosure is provided under "Use and Applications of the Device".

As used herein, the term "HIV" includes HIV-1 and HIV-2.

As used herein, the term "agent" includes any, including, but not limited to, any drug or prodrug, and encompasses, but is not limited to, traditional small molecule pharmaceuticals and biopharmaceuticals.

As used herein, the term "active pharmaceutical ingredient", "pharmaceutically active substance", "drug", "medicament", and "therapeutic agent" are used interchangeably.

As used herein, the term "API" means active pharmaceutical ingredient, which includes agents described herein.

The terms "drug delivery system" and "implant" are used interchangeably herein, unless otherwise indicated.

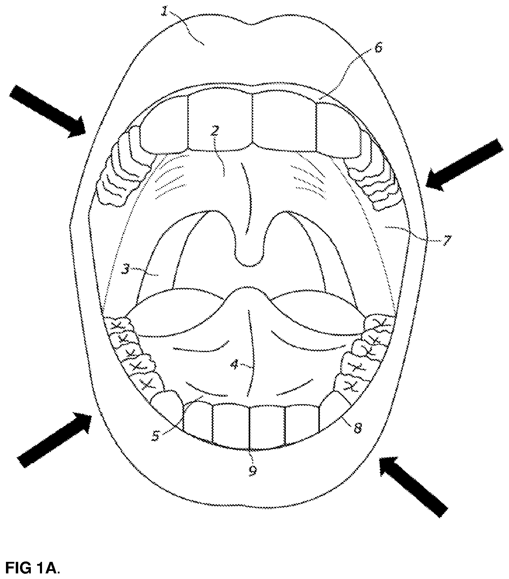

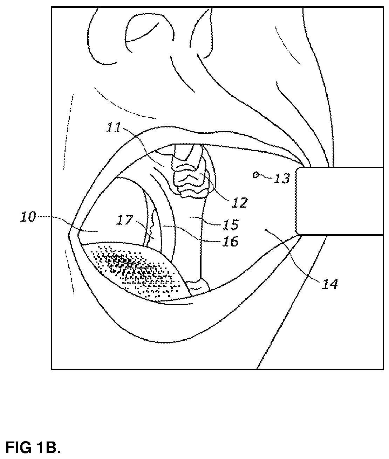

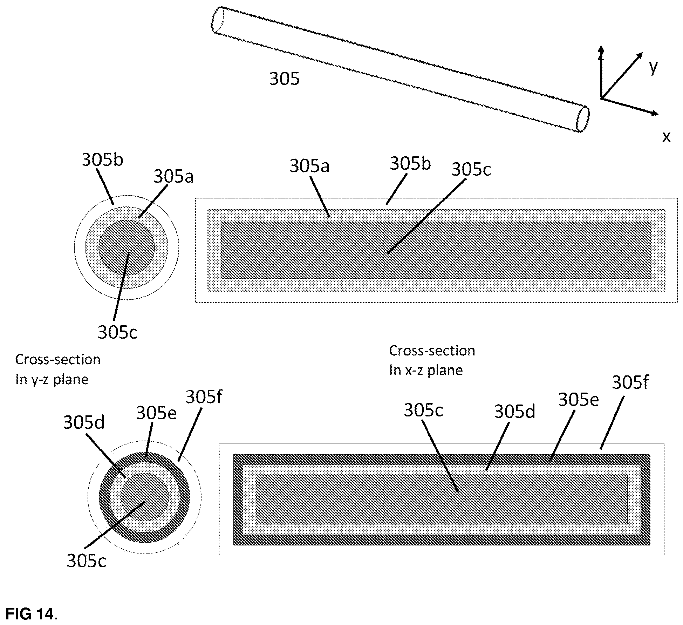

As used herein, the term "buccal" refers to the mucosa on the interior of the cheek (**7** in **FIG 1A; 14 in FIG 1B)** and connecting to the gingivae (both upper (maxillary) and lower (mandibular).

As used herein, the term "dental" refers to the outer surface of the teeth **(12** in **FIG 1B).**

As used herein, the term "proximal" refers to front of the oral cavity (i.e., towards the lips; **1** and **8** in **FIG 1A).**

As used herein, the term "distal" refers to refers to rear of the oral cavity (i.e., towards the throat; **10** in **FIG 1B).**

As used herein, the term "superior" refers to the upper area of the oral cavity (i.e., towards the nose).

As used herein, the term "inferior" refers to the lower area of the oral cavity (i.e., towards the chin).

"Matrix system" is defined as a system wherein a therapeutic agent is uniformly distributed in the matrix material and has no other release barrier than diffusion out of the matrix material.

"Reservoir" is the interior part of the drug delivery system containing the API and fully surrounded by a rate limiting skin.

"Kernel" is defined as one or more compartments that contain one or more APIs and makes up the majority of the device volume.

"Skin" means the outer portion of the drug delivery system that contacts the external environment. The skin can act as a rate limiting membrane depending of the composition of the skin.

"Rate limiting skin" is the part of the system which comprises polymer(s) with relatively low permeability for the therapeutic agents.

"Permeability" means the measurement of a therapeutic agent's ability to pass through a thermoplastic polymer.

"Reservoir system" means a drug delivery system that includes a drug-loaded reservoir, or kernel, surrounded by a skin and the diffusion of the drug through the skin is rate limiting. As a result of the rate limiting properties of the skin, an almost spatially uniform concentration of dissolved drug will be maintained in the kernel during release, whereas a concentration gradient will develop primarily in the skin.

"Mammal," as used herein, refers to any member of the class *Mammalia,* including, without limitation, humans and nonhuman primates such as chimpanzees and other apes and monkey species; farm animals such as cattle, sheep, pigs, goats and horses; domesticated mammals, such as dogs and cats; laboratory animals including rodents such as mice, rats and guinea pigs, and the like. The term does not denote a particular age or sex. Thus, adult and newborn subjects, whether male or female, are intended to be included within the scope of this term.

With the foregoing background in mind, in various embodiments, the disclosure teaches devices, systems and methods for treating, preventing, reducing the likelihood of having, reducing the severity of and/or slowing the progression of a condition in a subject.

**The Site of Implantation**

**Figure 1** schematically illustrates the oral cavity, and identifies terminologies used herein.

The oral cavity, and the buccal compartment in particular, is a microbe-rich and diverse body habitat identified in the Human Microbiome Project as one of the five key anatomic niches where the microbial taxa are key contributors to human health and disease (4). The diverse and abundant microorganisms inhabiting the oral mucosa are engaged in a dynamic, molecular dialog with their human hosts. These host-microbiome interactions also involve the host immune system leading to heightened surveillance and, hence, physiologic inflammation (normal/expected) of the buccal mucosa. Consequently, it would be non-obvious to one knowledgeable in the art to implant a foreign body, especially a drug delivery device designed to be in place for extended periods of time, in this microbe-dense and degradative enzyme-rich anatomic compartment.

## Implant Geometries

Devices for subcutaneous implantation such as the Implanon®/Explanon® contraceptive implants and Viadur® leuprolide acetate implant are typically of regular, cylindrical geometry. Regular geometric shapes can simplify implant manufacture; however, their geometry is not optimized to match the geometrical constraints of the implant location pertaining to the disclosed invention. The complexity of the oral cavity and buccal space geometry and its proximity to the jaw musculature makes it important for the implant geometry to fit the buccal space with minimal interference with jaw and other physical function along with little to no discomfort to the user. This requirement necessitates, in some embodiments, the employment of novel implant geometries specific to the buccal compartment. A number of geometries are suitable for buccal implant devices. In some embodiments, simple geometries based on regular three-dimensional shapes are desirable. Such shapes can simplify implant manufacture and, consequently, lower manufacturing cost.

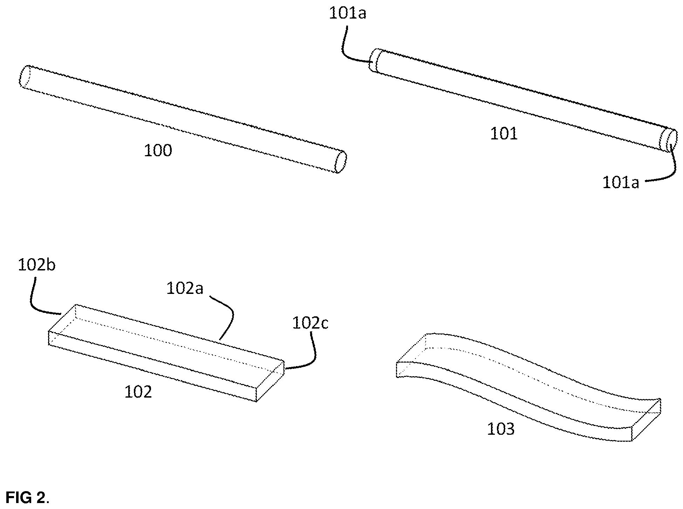

In one embodiment, the implant has a cylindrical or rod-shaped geometry, **100 (FIG 2).** Preferred lengths for rod-shaped implants are 5 - 50 mm, 5 - 10 mm, 10 - 20 mm, 20 - 30 mm, 30 - 40 mm, 40 - 50 mm, 10 mm, 15 mm, 20 mm, 25 mm, 30 mm, 35 mm, 40 mm, 45 mm, or 50 mm. Preferred rod diameters are 1 - 6 mm, 1 - 2 mm, 2 - 3 mm, 3 - 4 mm, 4 - 5 mm, 5 - 6 mm, 2 mm, 2.5 mm, 3 mm, 3.5 mm, 4 mm, 5 mm, or 6 mm. In other aspects of the disclosure, such as the embodiments described below, the implant has a non-cylindrical shape.

For example, in another embodiment, the implant has a rectangular prism geometry, **102 (FIG 2).** The rectangular geometry may be a thick sheet or a thin film. Rectangular prism geometries have a first dimension, **102a,** a second dimension, **102b,** and a third dimension, **102c.** For films, the shortest dimension **(102c)** can range from 0.01 - 1 mm, 0.01 - 0.1 mm, 0.1 - 0.5 mm, and 0.5 - 1 mm. In another embodiment, the shortest dimension **(102c)** of the rectangular geometry may be thicker, from 1 - 5 mm, 1 - 2 mm, 2 - 3 mm, 3 - 4 mm, or 4 - 5 mm. Preferred lengths for the longest dimension, **102a,** are 5 - 50 mm, 5 - 10 mm, 10 - 20 mm, 20 - 30 mm, 30 - 40 mm, 40 - 50 mm, 10 mm, 15 mm, 20 mm, 25 mm, 30 mm, 35 mm, 40 mm, 45 mm, or 50 mm. Preferred lengths for the intermediate length dimension, **102b,** are 1 - 10 mm, 1 - 2 mm, 2 - 3 mm, 3 - 4 mm, 4 - 5 mm, 5 - 6 mm, 6 - 7 mm, 7 - 8 mm, 8 - 9 mm, 9 - 10 mm, 2 mm, 2.5 mm, 3 mm, 3.5 mm, 4 mm, 5 mm, 6 mm, 7 mm, 8 mm, 9 mm, or 10 mm. In some embodiments, the faces of the implant are planar. One embodiment contains all planar faces, **102.** In other embodiments, one or more of the faces of the implant may be curved. One embodiment with curved faces is shown in **103.**

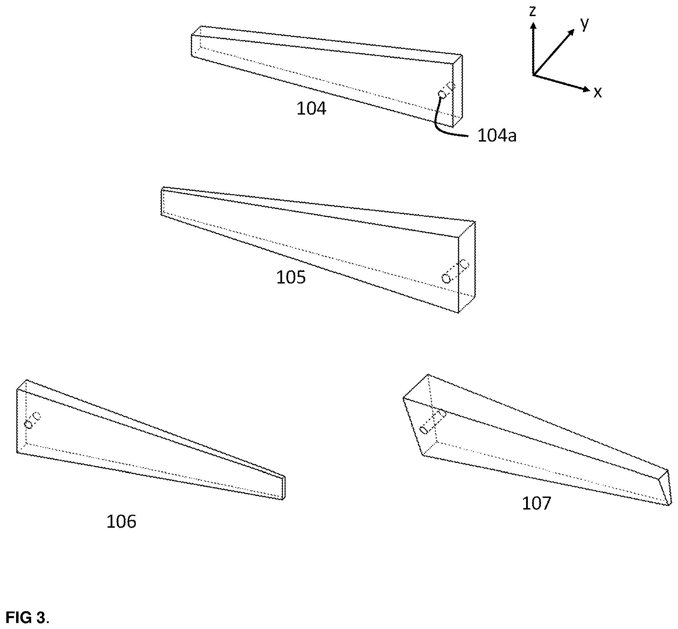

In another aspect, i.e., a simple rectangular prism embodiment, **102,** there are three pair of parallel faces. Other embodiments have one or more of the face pairs in a non-parallel arrangement to form an implant with one or more tapers. Tapers are formed when the angles formed by intersecting edges are not all 90°. In one embodiment, illustrated in **FIG 3,** the implant has a body **104** including a single taper formed by having two parallel face pairs and one face non-parallel, such that the body **104** tapers in the X-Z plane. In another embodiment, also illustrated in **FIG 3,** the implant has a body **105** that is tapered along two dimensions, such that the body **105** tapers in the X-Z and the X-Y planes. In another embodiment, also illustrated in **FIG 3,** the implant has a body **106** that is tapered in three dimensions, such that the body **106** has two tapers in the X-Z plane and one in the X-Y plane). In yet another embodiment, illustrated in **FIG 3,** the implant has a body **107** that tapers in the X-Z, X-Y, and Y-Z planes. In some cases, the buccal implant device is tapered as described above (i.e., the buccal implant device has a "saw shape"). In some cases, the buccal implant device has one, two, or three tapers. In some cases, the buccal implant device has one taper. In some cases, the buccal implant device has two tapers. In some cases, the buccal implant device has three tapers.

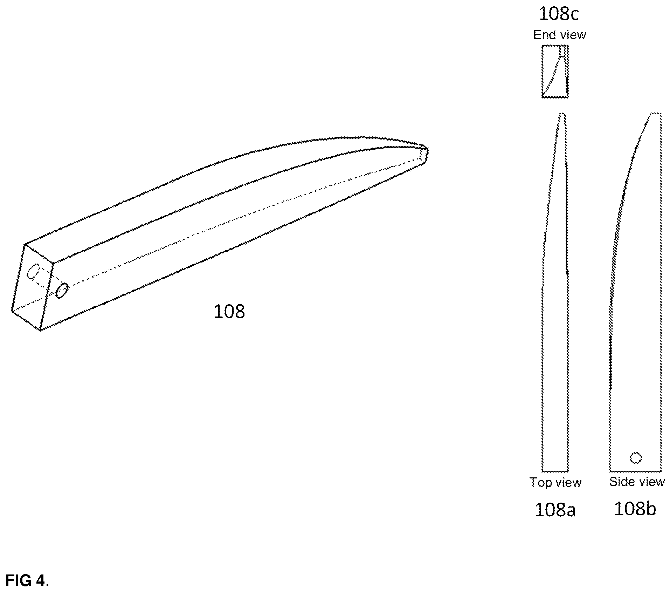

Other embodiments have curved edges to form implants with bodies having one or more curved faces **(FIG 4).** In one embodiment, the implant has a body **108** in which three faces are curved as shown in the two-dimensional projections of the top **(108a),** side **(108b),** and end **(108c)** views. The curved faces may be simply a preferred geometric design for aesthetic reasons, may simplify or lower the cost of manufacture, or may aid in the implantation procedure. In one embodiment, the curved edges form a point at the end of the implant. When the curved leading edge is inserted into a buccal vestibule (see "Methods for Implantation & Removal of the Device", below), the design enables lateral advancing by direct pressure on the distal end (end not yet inserted). The curved leading edge enhances separation of natural tissue planes with direct pressure. The curved edges can direct the implant into a preferred orientation. Any or all of the surfaces of the body of the implant may be curved, and the curvature may be concave as shown in **FIG 4** or convex. In some cases, the buccal implant device has one or more curved edges configured to direct the implant into a specific orientation within the oral cavity. In some cases, the one or more curved edges form a point at an end of the body. In some cases, the buccal implant device is curved as described above (i.e., the buccal implant device has a "saber shape").

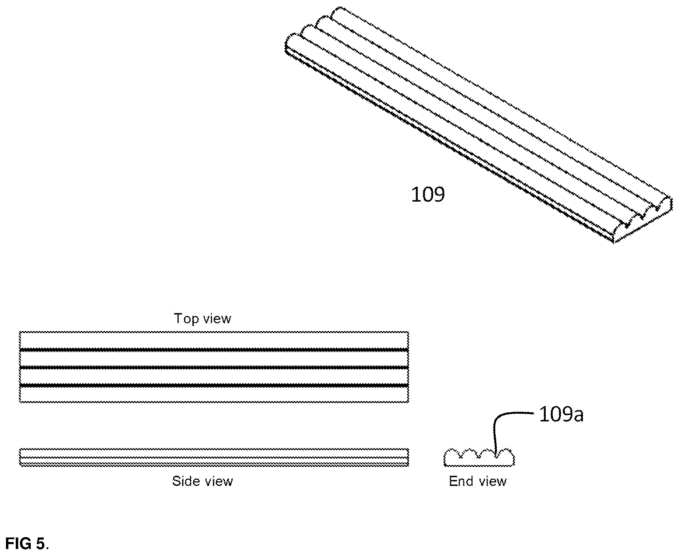

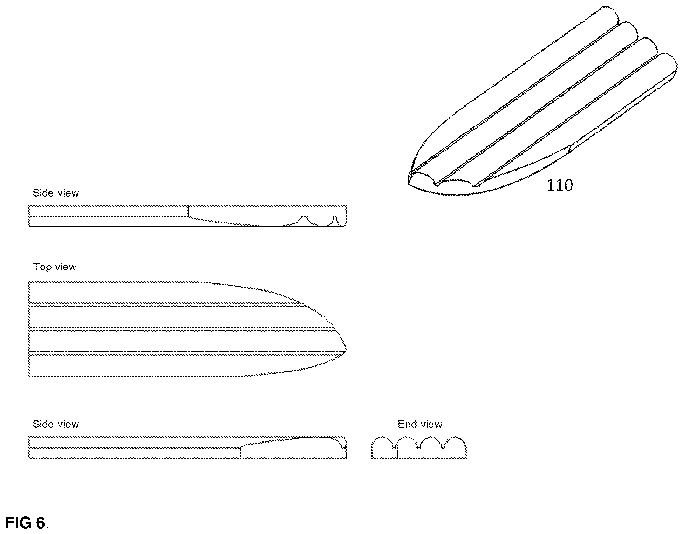

Another embodiment, illustrated in **FIG 5,** utilizes a ribbed implant geometry that resembles a series of two or more fused tubular lengths. In this embodiment, the implant has a body **109** having a corrugated or ribbed geometry that, e.g., minimizes resistance during implementation. The ribbed structure imparts additional flexibility along the longitudinal depressions, **109a,** and allows the device to bend or be rolled to aid insertion and to conform to the natural shape of the buccal cavity implantation site. The ribbed structure may also serve to retain the implant within the oral cavity implantation site. In another embodiment, illustrated in **FIG 6,** the implant has a body **110** that has this ribbed geometry and also includes curved edges, thereby combining the functionality of the bodies **108** and **109.** In some cases, the buccal implant device has a ribbed shape as described above.

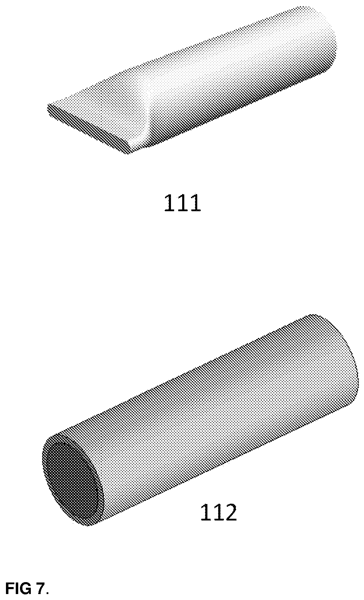

To aid in implant insertion and/or removal, a hole, **104a,** may be punched, molded, or otherwise formed in one end of the body of the implant. The hole may be used to grip the implant with forceps or another suitable tool. In some cases, the buccal implant device comprises a hole formed in the body. A loop made from suture material, wire, or other suitable material may be tied or otherwise attached to the hole to aid in gripping the implant for insertion and/or removal. In some cases, the buccal implant device comprises a loop coupled to the hole to guide the body into or out of the oral cavity. For implant designs with a kernel that is sealed in an outer shell or skin, two methods of sealing the shell may be employed. In one embodiment, one or both ends of the shell may be compressed and sealed **(FIG 7), 111.** In another embodiment, **112,** one or both ends of the implant are sealed using a plug to fill the empty space at the implant edge not filled by kernel material. Sealing shell material together in **111** or shell to plug in **112** may be accomplished by using an adhesive or by welding (melting) the material(s) to seal the joint (using, for example, a thermal induction welding, ultrasonic welding, or laser welding process).

In some embodiments, the buccal implant may include means for guiding the body of the implant into or out of the oral cavity. In one embodiment, the means for guiding the body may include a hole (e.g., the hole 104a). In another embodiment, the means for guiding the body may include a hole (e.g., the hole 104a) and a loop made from suture material, wire, or other suitable material coupled to the hole. In yet another embodiment, the means for guiding the body may be defined by one or more tapers of the body, such as, for example, illustrated in **FIG 3,** and/or one or more curved edges of the body, such as, for example, illustrated in **FIG 4.** In yet another embodiment, the means for guiding the body may include any of the foregoing.

In some embodiments, the buccal implant device has a shape or geometry which minimizes impediment of physical jaw function and/or discomfort to the patient. In some embodiments, the shape or geometry which minimizes impediment of physical jaw function and/or discomfort to the patient may include any of the foregoing.

**Implant Types**

There are three preferred, basic design types for the buccal implants disclosed herein, namely: (1) matrix; (2) reservoir; and (3) hybrid.

In some cases, the buccal implant device is of a matrix type. In some cases, the buccal implant device is of a reservoir type. In some cases, the buccal implant device is of a hybrid type.

*Matrix Designs*

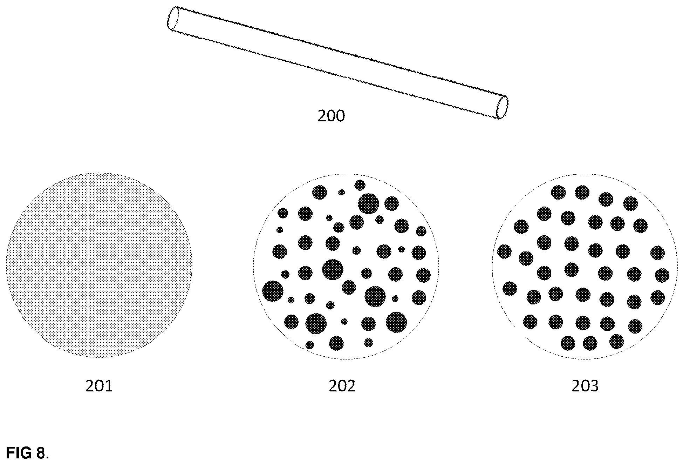

In one embodiment, the implant consists of a matrix-type design, **200 (FIG 8).** In the matrix design, the drug substance(s) is(are) distributed throughout the device, as a uniform solution in the elastomer, **201.** In another embodiment, the drug substance(s) is(are) distributed throughout the device in solid form as a suspension. As used herein, solid can include crystalline or amorphous forms. In one embodiment, the size distribution of the solid particles is polydisperse, **202.** In one embodiment, the size distribution of the solid particles is monodisperse, **203.** In one embodiment, the solid particles consist of nanoparticles (mean diameter < 100 nm). In one embodiment, the mean diameter of the particles is between 100 - 500 nm. Suitable mean particle diameters can range from 0.5 - 50 $\mu$m, from 0.5 - 5 $\mu$m, from 5 - 50 $\mu$m, from 1 - 10 $\mu$m, from 10 - 20 $\mu$m, from 20 - 30 $\mu$m, from 30 - 40 $\mu$m and from 40 - 50 $\mu$m. Other suitable mean particle diameters can range from 50 - 500 $\mu$m, from 50 - 100 $\mu$m, from 100 - 200 $\mu$m, from 200 - 300 $\mu$m, from 300 - 400 $\mu$m, and from 400 - 500 $\mu$m. Suitable particle shapes include spheres, needles, rhomboids, cubes, and irregular shapes.

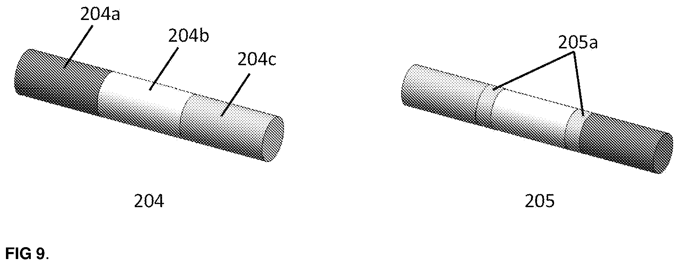

In one embodiment, the implant consists of a plurality of modules **(204a, 204b, 204c)** assembled into a single device **(FIG 9),** and each module is a matrix type component containing one or more drug substances. In one embodiment, the modules can be joined directly to one another (e.g., ultrasonic welding) or separated by an impermeable barrier, **205a,** to prevent drug diffusion between segments.

Matrix type implant devices are fabricated using methods known in the art. For example, an extrusion process can be used. Elastomer pellets cryomilled to a powder are blended with drug substance powder. Drug substance concentrations over a wide range, from 0.1-99% w/w, can be used with this approach. Alternatively, melt-mixing or extrusion compounding processes well known in the art can be used to blend drug substance with elastomer. The drug and polymer blends are hot-melt extruded to produce the implant drug product.

*Reservoir Designs*

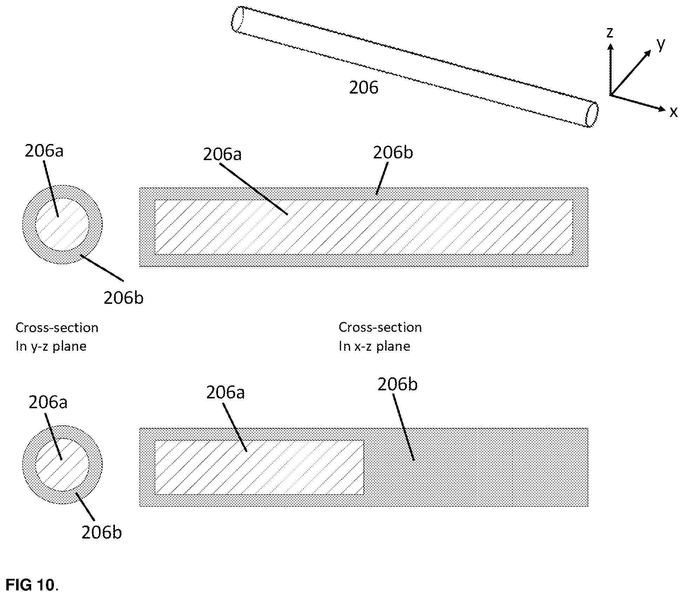

In one embodiment, the implant consists of a reservoir-type design, **206 (FIG 10).** In the reservoir implant, a kernel, **206a,** is loaded with the drug substance(s). The kernel can span the entire length of the device, or a partial length. The kernel is completely surrounded by a skin, **206b,** (described in more detail under "Hybrid Designs") that forms a barrier to drug diffusion, i.e., slows down the rate of release from the device. In one embodiment, a single skin material is employed. In another embodiment, one or more skin materials with different diffusion barrier properties may be used, each skin material covering a portion of the kernel so that the entire kernel surface is surrounded by a skin. Accordingly, the release of drug substances from such implants is dependent upon permeation (i.e., molecular dissolution and subsequent diffusion) of the kernel-loaded drug substance through the outer sheath, or skin. Drug release rates can be modified by changing the thickness of the rate-limiting skin. The drug release kinetics from reservoir type implants typically are zero to first order, depending on the characteristics of the kernel and skin.

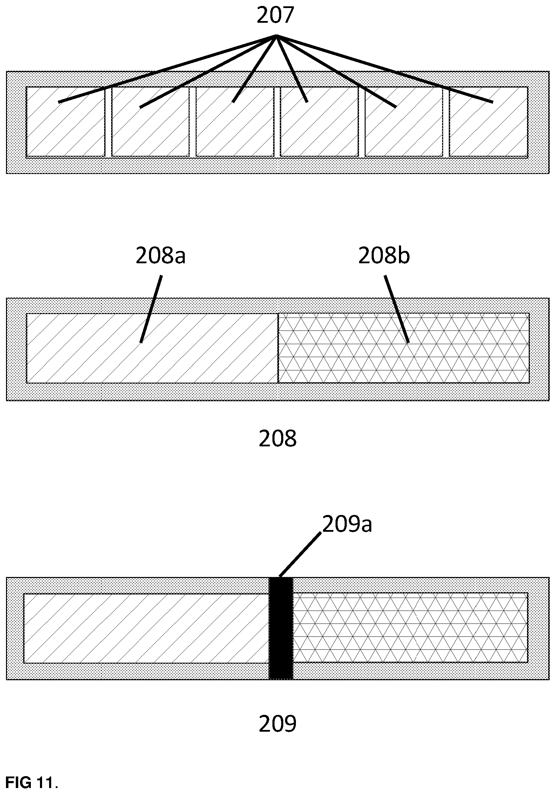

There are many embodiments describing the physical and chemical characteristics of the kernel. In one embodiment, the kernel consists of a powder made up of the API with or without excipients. In another embodiment, the kernel contains no API with the intent of have structural impact only. In another embodiment, the kernel consists of one or more pellets or microtablets, **207 (FIG 11),** as known in the art (5). In these embodiments, it may be desirable to maximize the drug loading and to minimize the use of excipients. However, the use of excipients can lead to beneficial physical properties such as lubrication and binding during tableting. Some excipients (e.g., vegetable, animal, and synthetics oils) can be used to create suspensions of the drug substance particles to form a paste, making the formulation easily dispensable into the implant shell, leading to manufacturing benefits. In some cases, the use of excipients can affect the solubility, and hence implant release rate, of the drug substance in the kernel. Certain excipients can be used to increase the solubility of drugs in water, and others can decrease the solubility. In some cases, excipients can lead to drug stabilization. Exemplary excipients are described in more detail below (see "Drug Formulation").

In some embodiments, multiple reservoir modules **(208a, 208b)** are joined to form a single implant, **208.** In some embodiments, **209,** the segments are separated by an impermeable barrier, **209a,** to prevent drug diffusion between segments.

In some embodiments, the skin is medicated with an API, as described under "Hybrid Designs".

*Hybrid Designs*

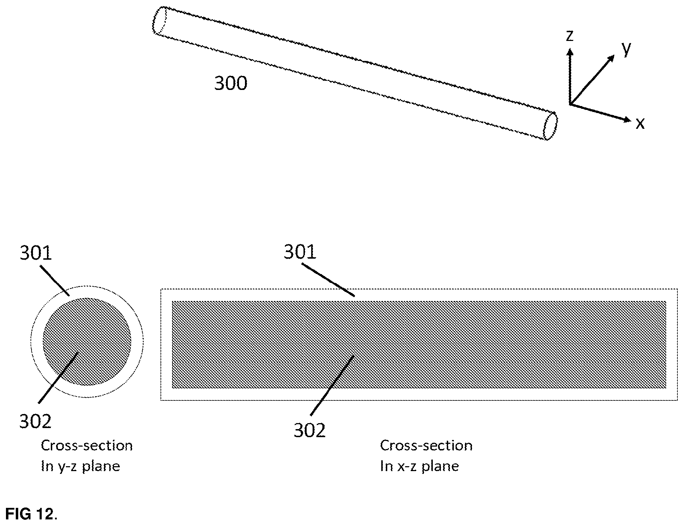

The *in vitro* and *in vivo* drug release profile of the matrix implants disclosed herein generally are non-linear, with an initial burst of drug release followed by a low, sustained release phase. In certain indications, it may be desirable to linearize the drug release properties of the implant. In an advantageous embodiment of such a buccal implant, the external surface of the device, **300,** is covered by a rate-limiting skin, **301 (FIG 12).** In one embodiment, the skin is made up of a biocompatible elastomer, as described in more detail under "Implant Materials" below. In one embodiment, the elastomer skin consists of a poly(ethylene-co-vinyl acetate), or ethylene vinyl acetate (EVA). The composition and thickness of the skin determines the extent of linearization of the drug release as well as the rate of drug release. The skin thickness can range from 5 - 700 $\mu$m and, for example, a vinyl acetate content ranging from 1 to 75%. The percentage vinyl acetate can be established using potentiometric titration as described in various textbooks on this subject matter. Suitable thicknesses of the skin can range from 5 - 700 $\mu$m, from 10 - 500 $\mu$m, from 15 - 450 $\mu$m, from 20 - 450 $\mu$m, from 30 - 400 $\mu$m, from 35 - 350 $\mu$m, and from 40 - 300 $\mu$m. In certain embodiments the thickness of the skin is 10 $\mu$m, 20 $\mu$m, 30 $\mu$m, 40 $\mu$m, 50 $\mu$m, 60 $\mu$m, 70 $\mu$m, 80 $\mu$m, 90 $\mu$m, 100 $\mu$m, 125 $\mu$m, 150 $\mu$m, 175 $\mu$m, 200 $\mu$m, 225 $\mu$m, 250 $\mu$m, and 300 $\mu$m. In some embodiments, the thickness of the skin is 30 $\mu$m, 50 $\mu$m or 80 $\mu$m. These skin characteristics also apply to reservoir-type designs.

In certain embodiments, the buccal implant drug delivery systems described herein comprising the first therapeutic agent in the kernel, **302,** and the second therapeutic agent in a skin, **301,** surrounding the kernel, can be described as a matrix type system wherein the first thermoplastic polymer of the kernel and the second thermoplastic polymer of the skin are the same polymer. In other embodiments, the kernel can be described as a reservoir-type system.

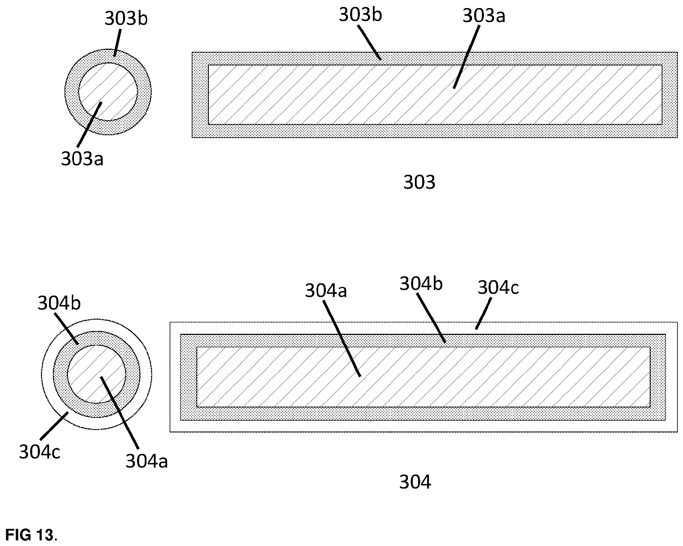

In other embodiments, **303 (FIG 13),** the buccal implant drug delivery systems described herein comprising the first therapeutic agent in the kernel, **303a,** (matrix or reservoir) and the second therapeutic agent in the skin, **303b,** surrounding the kernel, can be described as a hybrid between a reservoir type system and a matrix system or alternatively an enhanced matrix-type system, wherein the first thermoplastic polymer of the kernel and the second thermoplastic polymer of the skin are different polymers. The therapeutic agent loaded in the kernel will behave like a reservoir type system and will be released in a near zero-order fashion typical for reservoir systems, while the therapeutic agent loaded in the skin will exhibit a release profile more akin to a matrix-type system. It is this hybrid configuration that allows certain embodiments of the described buccal implant drug delivery systems to meet the necessary release criteria needed to achieve the desired therapeutic effect. In certain embodiments, of the buccal implant drug delivery systems described herein the second therapeutic agent is loaded into the skin such that the second therapeutic agent is dispersed uniformly though out the skin.

In other embodiments, **304 (FIG 13),** the therapeutic agent in a portion, or layer, of the skin, **304b,** and a second portion, or layer, of the skin served as a depletion layer, **304c.** A depletion layer is a is pre-formed or built-in portion of the skin that does not contain the second therapeutic agent. The depletion layer allows the therapeutic agent loaded in the remainder of the skin to display a near zero-order release profile, while obtaining higher release rates compared to a reservoir-type system. The depletion layer of the buccal implant drug delivery system described herein allows the second therapeutic agent in the skin to exhibit a near zero-order release profile while allowing the second therapeutic agent in the skin to meet the necessary release criteria needed to achieve a desired therapeutic effect.

Specifically, the second therapeutic agent in the skin, **304b,** will be released at a higher dose as compared to a drug release system wherein that same therapeutic agent had to pass through a drug-free rate limiting skin, **301.** The reason for this may be attributed to the fact that the drug incorporated in the skin only passes through the depletion layer instead of through the entire skin. Hence, the diffusion length is largely reduced but not entirely absent.

In certain embodiments, **303,** the skin, **303b,** comprises one layer. In other embodiments the skin comprises two layers. In still other embodiments, the skin comprises two or more layers, **304a** and **304b,** wherein, the second therapeutic agent is loaded in fewer than the totality of layers of the skin, for example, loaded in **304b** and not **304c,** creating a built-in depletion layer with the second therapeutic agent not completely dispersed throughout the entire skin.

For example, in certain embodiments of the buccal implant drug delivery system described herein, **305 (FIG 14),** the skin comprises a first layer, **305a,** and a second layer, **305b,** wherein the first layer is adjacent to the kernel (matrix or reservoir), **305c,** and the second layer is adjacent to the first layer. In certain embodiments, the first layer is loaded with the second therapeutic agent and the second layer contains no therapeutic agent and acts as the depletion layer.

In embodiments where the skin is medicated with a second API, the thickness of the skin is determined by the concentration of the second therapeutic agent and the desired release rate of the second therapeutic agent. Suitable thicknesses of the skin can range from 5 - 1000 $\mu$m, from 10 - 500 $\mu$m, from 15 - 450 $\mu$m, from 25 - 450 $\mu$m, from 35 - 400 $\mu$m, from 40 - 350 $\mu$m, and from 50 - 300 $\mu$m. In certain embodiments the thickness of the skin is 10 $\mu$m, 20 $\mu$m, 30 $\mu$m, 40 $\mu$m, 50 $\mu$m, 60 $\mu$m, 70 $\mu$m, 80 $\mu$m, 90 $\mu$m, 100 $\mu$m, 125 $\mu$m, 150 $\mu$m, 175 $\mu$m, 200 $\mu$m, 225 $\mu$m, 250 $\mu$m, 300 $\mu$m, 350 $\mu$m, 400 $\mu$m, 450 $\mu$m, or 500 $\mu$m.

In certain embodiments, the first layer and second layer of the skin are made from the same polymer. However, it can be envisioned that different polymers can be used for the first layer and second layer of the skin so long as the second therapeutic agent in the skin the necessary release criteria needed to achieve a desired therapeutic effect.

In other embodiments, the skin comprises three layers **(FIG 14),** a first layer, **305d,** adjacent to the kernel, a second layer, **305e,** adjacent to the first layer, and a third layer, **305f,** adjacent to the second layer. In one embodiment, the second layer is loaded with the second therapeutic agent and the third layer acts as the depletion layer. In another embodiment, one therapeutic agent is loaded in the first layer, the second layer serves as a depletion layer for the first layer, and the third layer is loaded with a second therapeutic agent. In another embodiment, all three layers are loaded with different therapeutic agents. In yet another set of embodiments, two or three of the layers contain the same therapeutic agent, but loaded at different concentrations in each layer.

In certain embodiments, the first, second and third layers of the skin are made from the same polymer. However, it can be envisioned that different polymers can be used for the first, second and third layers of the skin so long as the second therapeutic agent in the skin meets the necessary release criteria needed to achieve a desired therapeutic effect.

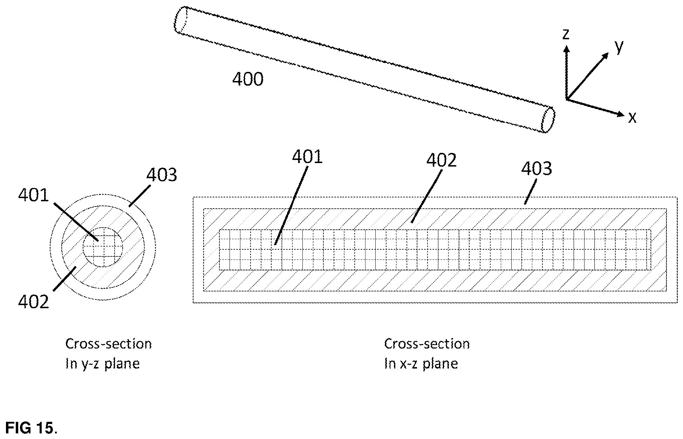

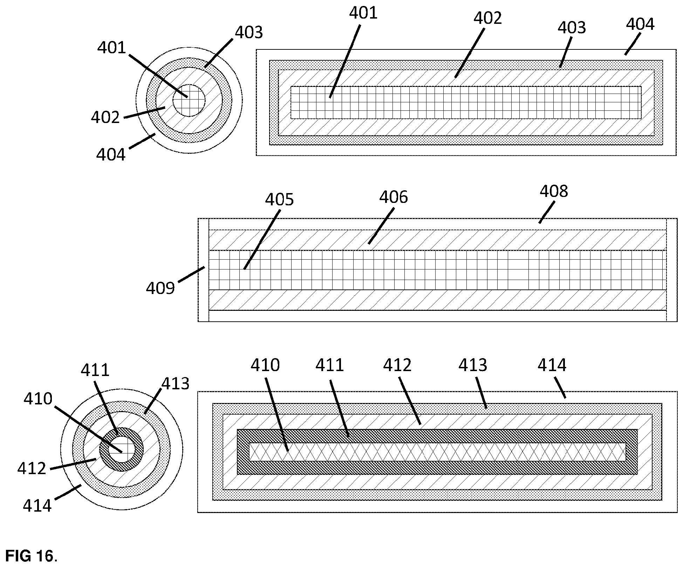

In certain embodiments described herein, the kernel of a matrix or reservoir implant can be a single compartment. In other embodiments, the kernel of the drug delivery systems described herein may be comprised of two compartments in a segmented arrangement as in **208** or arranged in two layers **(401, 402)** as in **400 (FIG 15).** In other embodiments, the kernel of the drug delivery systems described herein may be comprised of more than two compartments or layers. Each kernel layer may contain one or more therapeutic agents, or no therapeutic agents. For example, in certain embodiments of the buccal implant drug delivery systems described herein, the kernel is comprised of a first layer, **401,** and a second layer, **402,** wherein the second layer is adjacent to the skin, **403,** and the first layer is adjacent to the second layer. A second skin layer, **404 (FIG 16),** may optionally be present adjacent to the first skin layer. In certain embodiments, one or more skin layers may contain a therapeutic agent as described previously for embodiments **303, 304,** and **305.** In one embodiment, the first kernel layer, **401,** is completely surrounded by the second kernel layer, **402.** Only the second kernel layer is in contact with the first skin layer. In an alternate embodiment, the first kernel layer, **405,** is concentric with the second kernel layer, **406,** but a portion of the first kernel layer contacts the first skin layer, **409,** at the implant end. The first skin layer may be continuous around the entire implant, or it may be composed of a second material in the form of an end cap, **409,** that contacts the first kernel layer. In a further embodiment, the first kernel layer, **410,** is separated from the second kernel layer, **412,** by a barrier layer, **411,** that does not contain a therapeutic agent. Optional first, **413,** and second, **414,** skin layers may be present adjacent to the second kernel layer.

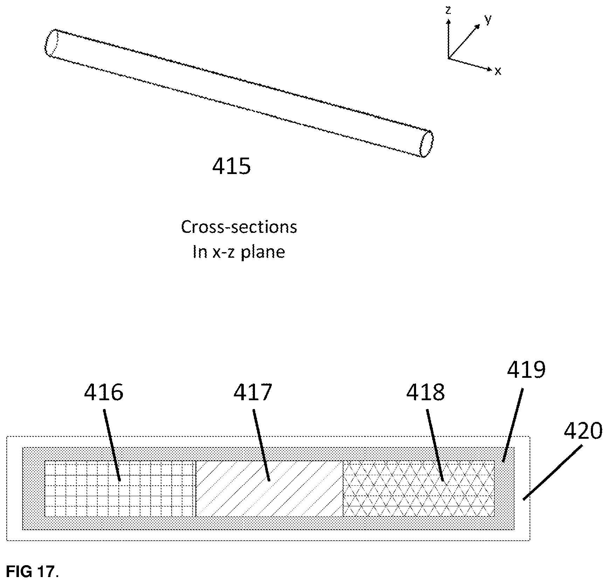

Another embodiment **(FIG** 17) has layers adjacent to one another along the implant long axis, **416, 417,** and **418,** with a first skin layer, **419,** and optionally a second skin layer, **420.**

In certain embodiments, all layers of the kernel are made from the same polymer. However, it can be envisioned that different polymers can be used for one or more of the kernel layers.

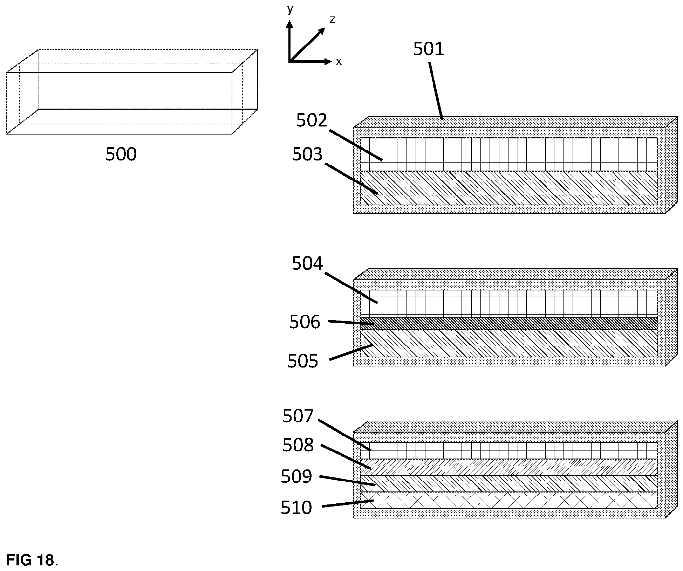

In yet another set of embodiments, **500 (FIG 18),** the kernel is composed of one or more stacked layers. In a preferred embodiment, there are two stacked kernel layers, **502** and **503,** surrounded by a one or more skin layers, **501.** In a second preferred embodiment, kernel layers **504** and **505** are separated by a non-medicated barrier layer, **506.** In yet a third embodiment, more than two **(507 - 510)** stacked layers are employed, either with or without a separating barrier layer.

In certain embodiments of the buccal implant drug delivery devices described herein, a buccal implant drug delivery device comprises: (a) a kernel (matrix or reservoir) comprising a first therapeutic agent; a first thermoplastic polymer; a first kernel layer and a second kernel layer; and (b) a skin surrounding the kernel comprising a second therapeutic agent; a second thermoplastic polymer; a first skin layer and a second skin layer wherein the first skin layer is between the second skin layer and the second kernel layer.

In other embodiments, the kernel (matrix or reservoir) comprises three layers, a third layer adjacent to the skin, a second layer adjacent to the third layer and a first layer adjacent to the second layer.

In certain embodiments, the first, second and third layers of the kernel (matrix or reservoir) are made from the same polymer. However, it can be envisioned that different polymers can be used for the first, second and third layers of the kernel so long as the first therapeutic agent in the kernel experiences a reduced permeation resistance as it is being released through the skin and meets the necessary release criteria needed to achieve a desired therapeutic effect.

In certain embodiments, the first therapeutic agent is in dissolved form in the kernel and the second therapeutic agent is in solid form in the skin. As used herein, the term solid form can include crystalline or amorphous forms. In certain embodiments, the first therapeutic agent is in solid form in the kernel and the second therapeutic agent is in solid form in the skin. In certain embodiments, the first therapeutic agent is in solid form in the kernel and the second therapeutic agent is in dissolved form in the skin. In certain embodiments, the first therapeutic agent is in the kernel of a reservoir-type system and the second therapeutic agent is in solid form in the skin. As used herein, the term solid form can include crystalline or amorphous forms. In certain embodiments, the first therapeutic agent is in the kernel of a reservoir-type system and the second therapeutic agent is in dissolved form in the skin.

## Implant Materials

In one embodiment, the buccal implants disclosed herein are composed of one or more suitable thermoplastic polymers, elastomer materials, or metals suitable for pharmaceutical use. Such materials are known in the art, and described in the literature (6, 7).

In one embodiment, the implant (kernel and/or skin) elastomeric material is non-resorbable. It may be composed of a medical-grade poly(dimethyl siloxane) or silicone, as known in the art. Other examples of suitable non-resorbable materials include: synthetic polymers selected from poly(ethers); poly(acrylates); poly(methacrylates); poly(vinyl pyrolidones); poly(vinyl acetates), including but not limited to EVA, poly(urethanes); celluloses; cellulose acetates; poly(siloxanes); poly(ethylene); poly(tetrafluoroethylene) and other fluorinated polymers; poly(siloxanes); copolymers thereof and combinations thereof. The implant may also consist of a biocompatible metal such as titanium, stainless steel, and others known in the art.

In another embodiment, the implant (kernel and/or skin) elastomeric material is resorbable. In one embodiment of a resorbable device, the skin is formed of a biodegradable or bioerodible polymer. Examples of suitable resorbable materials include: synthetic polymers selected from poly(amides); poly(esters); poly(ester amides); poly(anhydrides); poly(orthoesters); polyphosphazenes; pseudo poly(amino acids); poly(glycerol-sebacate); copolymers thereof, and mixtures thereof. In one embodiment, the resorbable synthetic polymers are selected from poly(lactic acids), poly(glycolic acids), poly(lactic-co-glycolic acids), poly(caprolactones) (PCLs), and mixtures thereof. Other curable bioresorbable elastomers include PCL derivatives, amino alcohol-based poly(ester amides) (PEA) and poly(octane-diol citrate) (POC). PCL-based polymers may require additional cross-linking agents such as lysine diisocyanate or 2,2-bis(-caprolacton-4-yl) propane to obtain elastomeric properties.

In one embodiment of the buccal implant drug delivery systems described herein (kernel and/or skin), the elastomeric material consists of suitable thermoplastic polymer or elastomer material that can, in principle, be any thermoplastic polymer or elastomer material suitable for pharmaceutical use, such as silicone, low density polyethylene, EVA, polyurethanes, and styrene-butadiene-styrene copolymers.

In certain embodiments, EVA is used in the kernel and the skin due to its excellent mechanical and physical properties. The EVA material may be used for the kernel, as well as the skin and can be any commercially available EVA, such as the products available under the trade names: Elvax, Evatane, Lupolen, Movriton, Ultrathene and Vestypar.

The permeability of EVA copolymers for small to medium sized drug molecules ($M \leq 600$ g mol$^{-1}$) is primarily determined by the vinyl acetate to ethylene ratio. Low-VA content EVA copolymers are substantially less permeable than high VA-content skins and hence display rate limiting properties if used as skin. EVA copolymers with VA-content of 19% w/w or less ($\leq 19\%$ w/w) are substantially less permeable than polymer having VA-content above and including 25% w/w (> 25% w/w).

In some embodiments, the first thermoplastic polymer is an EVA and has a vinyl acetate content of 28% or greater. In other embodiments, the first thermoplastic polymer has a vinyl acetate content of greater than 28%. In still other embodiments, the first thermoplastic polymer has a vinyl acetate content between 28-40% vinyl acetate. In yet other embodiments, the first thermoplastic polymer has a vinyl acetate content between 28-33% vinyl acetate. In one embodiment, the first thermoplastic polymer has a vinyl acetate content of 28%. In one embodiment, the first thermoplastic polymer has a vinyl acetate content of 33%. In some embodiments, the second thermoplastic polymer is an ethylene-vinyl acetate copolymer and has a vinyl acetate content of 28% or greater. In other embodiments, the second thermoplastic polymer has a vinyl acetate content of greater than 28%. In still other embodiments, the second thermoplastic polymer has a vinyl acetate content between 28-40% vinyl acetate. In yet other embodiments, the second thermoplastic polymer has a vinyl acetate content between 28-33% vinyl acetate. In one embodiment, the second thermoplastic polymer has a vinyl acetate content of 28%. In one embodiment, the second thermoplastic polymer has a vinyl acetate content of 33%.

In some embodiments, the second thermoplastic polymer is an EVA and has a vinyl acetate content of 28% or less. In other embodiments, the second thermoplastic polymer has a vinyl acetate content of less than 28%. In still other embodiments, the second thermoplastic polymer has a vinyl acetate content between 9-28% vinyl acetate. In yet other embodiments, the second thermoplastic polymer has a vinyl acetate content between 9-18% vinyl acetate. In one embodiment, the second thermoplastic polymer has a vinyl acetate content of 15%. In one embodiment, the second thermoplastic polymer has a vinyl acetate content of 18%.

It should be noted that when a specific vinyl acetate content, e.g., 15%, is mentioned, it refers to the manufacture's target content, and the actual vinyl acetate content may vary from the target content, e.g., by plus or minus 1% or 2%. One of ordinary skill in the art would appreciate that suppliers may use internal analytical methods for determining vinyl acetate content, thus there may be an offset between methods.

## Formulations

*Target* in Vivo *Drug Release Kinetics and Profiles*

The drug formulation may provide a temporally modulated release profile or a more continuous or consistent release profile.

Pulsatile release can be achieved from a plurality of devices, implanted simultaneously or in a staggered fashion over time. The implants can be inserted in superior or inferior regions of the buccal mucosa, as indicated in **FIG 1.** In another embodiment, pulsatile drug release can be achieved from a single device wherein a plurality of kernels is separated by biodegradable skins or barriers. As they erode over time, the degradable skins can be used to temporally stagger the release of one or more agents from each of several kernels. This approach is compatible with the various implant geometries and designs disclosed herein.

*Choice of API*

The drug formulation can include essentially any therapeutic, prophylactic, or diagnostic agent that would be useful for buccal delivery. The buccal implant drug delivery devices according to the disclosure comprise at least one pharmaceutically active substance, including, but not limited to, agents that are used in the art for the applications described under "Use and Applications of the Device", and combinations thereof. In one embodiment, the drug delivery device comprises two or more pharmaceutically active substances. In this instance, the pharmaceutically active substances can have the same hydrophilicity or hydrophobicity or different hydrophilicities or hydrophobicities. In some cases, the buccal implant device (or any sustained release device described herein) comprises one or more active pharmaceutical ingredients selected from antiretrovirals, antimicrobial agents, antibacterial agents, antivirals, hormones, statins, β-blockers, ACE inhibitors, angiotensin receptor blockers, vitamins, steroids, biologics, anti-cancer drugs, allergy medications, anticoagulants, antiplatelet therapies, non-steroidal anti-inflammatory drugs, vaccines, and combinations thereof.

Nonlimiting examples of hydrophobic pharmaceutically active substances include: cabotegravir, dapivirine, fluticasone propionate, chlordiazepoxide, haloperidol, indomethacin, prednisone, and ethinyl estradiol. Nonlimiting examples of hydrophilic pharmaceutically active substances include: acyclovir, tenofovir, atenolol, aminoglycosides, exenatide acetate, leuprolide acetate, acetylsalicylic acid (aspirin), and levodopa. In some cases, the buccal implant device (or any sustained release device described herein) comprises one or more active pharmaceutical ingredients comprise atazanavir, didanosine, efavirenz, emtricitabine, lamivudine, lopinavir, nevirapine, raltegravir, ritonavir, saquinavir, stavudine, tenofovir, tenofovir disoproxil fumarate, tenofovir alafenamide, zidovudine, acyclovir, famciclovir, valcyclovir, morphine, buprenorphine, estrogen, progestin, progesterone, cyclosporine, a calcineurin inhibitor, prostaglandin, cabotegravir, rilpivirine, lenacapavir (GS-6207), 4'-ethynyl-2-fluoro-2'-deoxyadenosine (MK-8591, EFdA), a beta-blocker, gentamycin, corticosteroid, a fluoroquinolone, insulin, an antineoplastic drug, anti-nausea drug, a corticosteroid, an antibiotic, morphine buprenorphine, a VEGF inhibitor, or combinations thereof. In some cases, the buccal implant device (or any sustained release device described herein) comprises tenofovir. In some cases, the buccal implant device comprises tenofovir alafenamide ("TAF").

In some cases, the pharmaceutically active substance is chloroquine or hydroxychloroquine, pharmaceutically acceptable salts thereof, or combinations thereof. In some cases, the pharmaceutically acceptable salt is a phosphate, such as a diphosphate, or a chloride, such as a dichloride, or combinations thereof.

In some cases, the pharmaceutically active substance is an antibacterial agent. In some cases, the antibacterial agent is a broad-spectrum antibacterial agent. Non-limiting examples of antibacterial agents include azithromycin.

In some cases, the pharmaceutically active substance is an antiviral agent. Non-limiting examples of antiviral agents include remdesivir (Gilead Sciences), acyclovir, ganciclovir, and ribavirin, and combinations thereof. In some cases, the pharmaceutically active substance is an antiretroviral drug. In some cases, the antiretroviral drug is used to treat HIV/AIDS. Non-limiting examples of antiretroviral drugs include protease inhibitors.

In some cases, the pharmaceutically active substance is an agent that affects immune and fibrotic processes. Non-limiting examples of agents that affect immune and fibrotic processes include inhibitors of Rho-associated coiled-coil kinase 2 (ROCK2), for example, KD025 (Kadmon).

In some cases, the pharmaceutically active substance is a sirtuin (SIRT1-7) inhibitor. In some cases, the sirtuin inhibitor is EV-100, EV-200, EV-300, or EV-400 (Evrys Bio). In some cases, administration of a sirtuin inhibitor restores a human host's cellular metabolism and immunity.

The pharmaceutically active substances described herein can be administered alone or in combination. Combinations of pharmaceutically active substances can be administered using one implant or multiple implants. In some cases, the implants described here comprise one pharmaceutically active substance. In some cases, the implants described herein comprise more than one pharmaceutically active substance. In some cases, the implants described herein comprise a combination of pharmaceutically active substances. In some cases, the combination of pharmaceutically active substances is chloroquine and azithromycin, hydroxychloroquine and azithromycin, lopinavir and ritonavir, KD025 and ribavirin, KD025 and remdesivir, EV-100 and ribavirin, or EV-100 and remdesivir.

The suitability of any given pharmaceutically active substance in the context of the disclosure is not limited or predicated by any given medical application, but rather is a function of the following non-limiting parameters:

*Potency;* the potency of the API will determine whether it can be formulated into one or more buccal implants and maintain pharmacologically relevant concentrations in the key anatomic compartment(s) for the target duration of use (see "Example 1"),

*Implant Payload;* the amount of API that can be formulated into a buccal implant of choice, and the number of feasible devices implanted at one time, together with the API potency is a primary limiting factor in selecting an API for a given application (see "Example 1" and "Example 2"),

*Solubility;* the aqueous solubility of the API must be such that delivery *via* implant is achievable at the target rate. The solubility, and hence release rate, of the API also can be modulated (increased or decreased) using suitable excipients as known in the art,

*Targeted Delivery;* buccal drug delivery, as disclosed herein, targets the systemic circulatory system,

*Local Toxicity;* the systemic toxicity profile of many APIs envisioned in the disclosed application will have been determined prior to formulation into buccal implants. Local toxicity at the implantation site therefore represent the largest safety concern in these cases, and could limit the API delivery rate. In some cases, drugs have a low therapeutic index (TI) and it may not be possible to control the drug release rate from the implant to provide safe and effective concentrations,

*Cost*; the API cost could be limiting in certain cases.

*In silico* prediction of implant specifications for any given API and medical application and the development of a Target Product Profile (TPP) is highly challenging, as known in the art for other sustained release drug delivery technologies, and requires preclinical studies followed by clinical validation of the pharmacology in terms of pharmacokinetics (PK) and pharmacodynamics (PD, safety and efficacy).

*API Formulation*

The API formulation may consist only of the drug, or may include one or more agents and/or one or more pharmaceutically acceptable excipients. Pharmaceutically acceptable excipients are known in the art and may include: viscosity modifiers, bulking agents, surface active agents, dispersants, disintegrants, osmotic agents, diluents, binders, anti-adherents, lubricants, glidants, pH modifiers, antioxidants and preservants, and other non-active ingredients of the formulation intended to facilitate handling and/or release kinetics of the drug. In some cases, the buccal implant device (or any sustained release device described herein) comprises one or more pharmaceutically acceptable excipients. In some cases, the pharmaceutically acceptable excipient is present in an amount of about 20% w/w to about 30% w/w. In some cases, the pharmaceutically acceptable excipient is triethyl citrate (TEC) or PEG 400. In some cases, the pharmaceutically acceptable excipient is triethyl citrate (TEC). In some cases, the pharmaceutically acceptable excipient is PEG 400.

In some embodiments, the binders and/or disintegrants may include, but are in no way limited to, starches, gelatins, carboxymethylcellulose, croscarmellose sodium, methyl cellulose, ethyl cellulose, hydroxymethyl cellulose, hydroxyethyl cellulose, hydroxypropyl cellulose, hydroxypropylmethyl cellulose, hydroxypropylethyl cellulose, hydroxypropylmethyl cellulose, microcrystalline cellulose, polyvinylpyrrolidone, polyethylene glycol, sodium starch glycolate, lactose, sucrose, glucose, glycogen, propylene glycol, glycerol, sorbitol, polysorbates, and colloidal silicon dioxide. In certain embodiments, the anti-adherents or lubricants may include, but are in no way limited to, magnesium stearate, stearic acid, sodium stearyl fumarate, and sodium behenate. In some embodiments, the glidants may include, but are in no way limited to, fumed silica, talc, and magnesium carbonate. In some embodiments, the pH modifiers may include, but are in no way limited to, citric acid, lactic acid, and gluconic acid. In some embodiments, the antioxidants and preservants may include, but are in no way limited to ascorbic acid, butylated hydroxytoluene (BHT), butylated hydroxyanisole (BHA), cysteine, methionine, vitamin A, vitamin E, sodium benzoate, and parabens.

*Foreign Body Response*

Silicone implants, for example, are inexpensive and wieldy, but may elicit a foreign-body reaction and can be prone to migration. Gore-Tex (expanded polytetrafluoroethylene, ePTFE) implants are more biocompatible and capable of ingrowth, but expensive and may block drug release. Silicone-ePTFE composites have a silicone core and PTFE liner and are used in surgical applications, such as rhinoplasty (*10, 11*) and cheek-lip groove rejuvenation (*12*).

In one embodiment, the outermost elastomer implant skin is bonded to an outer ePTFE sleeve to form a composite (i.e., the ePTFE sleeve only serves to mitigate the foreign body response and does not control or affect drug release from the device).

It is known in the art that the host's foreign body response can affect the safety of an implanted device, particularly for subcutaneous implants (13), or other types of devices implanted into an otherwise sterile area or compartment. This reaction consists of protein adsorption on the implant surface, inflammatory cell infiltration, macrophage fusion into foreign body giant cells, fibroblast activation and ultimately fibrous encapsulation. This series of events may affect the function of subcutaneous implants, such as inhibition of drug diffusion from long-acting drug delivery depots and medical device failure. To date, combination approaches, such as hydrophilic coatings that reduce protein adsorption combined with delivery of dexamethasone are the most effective.

In a particular embodiment, the implantable drug delivery device releases one or more agents to mitigate or reduce the foreign body response in addition to the primary API. These agents are mixed with the API and any excipients, and formulated into the drug reservoir (see "Drug Formulation", below). The agents are released from the implant with the API. In one embodiment, the agent included to reduce the foreign body response is a steroid. In one embodiment, this steroid is dexamethasone, or a dexamethasone derivative such as dexamethasone 21-acetate or dexamethasone 21-phosphate disodium salt.

It is known in the art that hydrogels, particularly zwitterionic hydrogels, can significantly reduce the foreign body response to subdermal implants (14).

**Exemplary Target Implant Specifications**

The amount of pharmaceutically active substance(s) incorporated into the buccal implant can also be calculated as a pharmaceutically effective amount, where the devices of the present implants comprise a pharmaceutically effective amount of one or more pharmaceutically active substances. By "pharmaceutically effective", it is meant an amount that is sufficient to elicit the desired physiological or pharmacological change in subject. This amount will vary depending upon such factors as the potency of the particular pharmaceutically active substance, the density of the pharmaceutically active substance, the shape of the implant, the desired physiological or pharmacological effect, and the time span of the intended treatment. Those skilled in the pharmaceutical arts will be able to determine the pharmaceutically effective amount for any given pharmaceutically active substance in accordance with the standard procedure.

In some embodiments, the pharmaceutically active substance is present in an amount ranging from about 1 mg to about 3,000 mg of pharmaceutically active substance per buccal implant. This includes embodiments in which the amount ranges from about 2 mg to about 25 mg, from about 25 mg to about 250 mg, and from about 250 mg to about 3,000 mg of pharmaceutically active substance per buccal implant.

In certain embodiments of the buccal implant drug delivery device described herein, wherein the first therapeutic agent is present in the kernel about 0.1%-99% w/w. In other embodiments, the first therapeutic agent is present in the kernel at about 65-100% w/w, at about 65-75% w/w, or at about 75-85% w/w, or about 85-99% w/w.

In some embodiments, the active pharmaceutical ingredient is released from the buccal implant device over a defined period of time. In certain embodiments, the buccal implant drug delivery systems described herein are capable of releasing the therapeutic agent(s) contained therein over a period of 3, 4, 5, or 6 weeks. In certain embodiments, the buccal implant drug delivery systems described herein are capable of releasing the therapeutic agent(s) contained therein over a period of 8, 10, 12 or 14 weeks. In certain embodiments, the buccal implant drug delivery systems described herein are capable of releasing the therapeutic agents contained therein over a period of 1, 2, 3, or 6 months. In some embodiments, the active pharmaceutical ingredient is released over a period of 1 to 12 months. In some embodiments, the active pharmaceutical ingredient is released over a period of 3 months. In certain embodiments, the buccal implant drug delivery systems described herein are capable of releasing the therapeutic agents contained therein over a period of one, two, three, or four years.

In one embodiment, the buccal implant drug delivery systems described herein, is capable of releasing tenofovir alafenamide (TAF), or its pharmaceutically acceptable salts, over a period of 3, 4, 6, or six weeks or 8, 10, 12 or 14 weeks, or 1, 2, 3, 6, or 12 months at an average rate of between 0.05-3 mg d$^{-1}$. In certain embodiments, the buccal implant described herein is capable of releasing TAF, or its pharmaceutically acceptable salts, at an average rate of between 0.1-2 mg d$^{-1}$. In certain embodiments, the buccal implant described herein is capable of releasing TAF, or its pharmaceutically acceptable salts, over a period of 3, 6, or 12 months at a rate of between 0.1-1 mg d$^{-1}$. In certain embodiments, the buccal implant described herein is capable of releasing TAF, or its pharmaceutically acceptable salts, at an average rate of 0.25 mg d$^{-1}$. In certain embodiments, the buccal implant described herein is capable of releasing TAF, or its pharmaceutically acceptable salts, at an average rate of 0.5 mg d$^{-1}$. In certain embodiments, the buccal implant described herein is capable of releasing TAF, or its pharmaceutically acceptable salts, at an average of 1 mg d$^{-1}$.

In certain embodiments of the buccal implant drug delivery device described herein, the second therapeutic agent is present in the skin at about 5-50% w/w. In other embodiments, the second therapeutic agent is present in the skin at about 10-50% w/w, at about 20-50% w/w, at about 10%, 30% or 50% w/w of the skin.

In certain embodiments, the buccal implant drug delivery systems described herein are stable at room temperature. As used herein, "room temperature" lies anywhere between about 18°C and about 30°C. As used herein, a physically stable buccal implant drug delivery system is a system that can be stored at about 18-30°C for at least about one month.

**Implant Fabrication**

Also described herein are methods of manufacturing the buccal implant drug delivery systems. In one embodiment, the implants are manufactured under fully aseptic conditions. In another embodiment, the implants are terminally sterilized using methods known in the art such as gamma sterilization, steam sterilization, dry heat sterilization, UV irradiation, ethylene oxide sterilization, and the like.

*Matrix and Hybrid Implant Fabrication*

Also described herein are methods of manufacturing the matrix and hybrid design buccal implant drug delivery systems described herein comprising:

Producing a homogenous polymer granulate kernel comprising the first therapeutic agent and a loaded skin layer granulate comprising the second therapeutic agent,

Co-extruding the kernel granulate comprising the first therapeutic agent and the skin layer granulate comprising the second therapeutic agent to form two-layered drug delivery system or co-extruding the kernel granulate comprising the first therapeutic agent with additional kernel layers and/or the skin granulate comprising the second therapeutic agent with additional skin layers to form a multi-layered drug delivery system.

Also described herein are methods of manufacturing the drug loaded kernel or skin granulate:

Grinding the polymer,
Dry powder mixing the ground polymer with the respective active compound,
Blend-extruding the resulting powder mixtures of Step (b),
Cutting the resulting loaded polymer strands into granules, thereby obtaining a kernel granulate and/or the skin layer granulate,
When required lubricating the granulate prior to coextrusion.

*Reservoir and Hybrid Implant Fabrication*

Also described herein are methods of manufacturing the buccal implant drug delivery systems of the reservoir design.

In one embodiment of reservoir-type buccal implants, the API, and any other solid agents or excipients, can be filled into the implant shell as a powder, paste, or slurry using filling methods known in the art. In another embodiment, the solid actives and carriers can be compressed into microtablet/tablet form to maximize the loading of the actives (5, 8), using means common in the art.

In one example, the drug formulation is in the form of a solid drug rod. Embodiments of drug rods, and methods of making such drug rods, are described in the art, such as (15). The drug rods may be formed by adapting other extrusion or casting techniques known in the art. For example, a drug rod comprising an API may be formed by filling a tube with an aqueous solution of the API and then allowing the solution to evaporate. As another example, a drug rod comprising of an API may be formed by extrusion, as known in the art. In many embodiments, the drug formulation desirably includes no or a minimum quantity of excipient for the same reasons of volume/size minimization.

Open ends of the implant can be closed with a pre-manufactured end plug to ensure a smooth end and a solid seal. Plugs may be sealed in the implant end using frictional force (for example, a rim and groove that lock together to form a seal); an adhesive; induction, ultrasonic, or laser welding, or another form of heat sealing that melts together the plug and implant end. In another embodiment, the ends are sealed without using a solid plug by one of a number of methods known to one skilled in the art, including but not limited to, heat-sealing, induction welding, ultrasonic welding, laser welding, or sealing with an adhesive.

In some embodiments, the buccal implant device comprises one or more sealed tubes. In some embodiments, the buccal implant device comprises one sealed tube. In some embodiments, the sealed tube has an inner diameter of about 1 mm to about 3 mm. In some embodiments, the sealed tube has an inner diameter of about 1.5, 2, 2.5, or 3 mm. For example, the sealed tube optionally has an inner diameter of about 2 mm. In some embodiments, the sealed tube has a wall thickness of about 0.1 to 0.3 mm. In some embodiments, the sealed tube has a wall thickness of about 0.1, 0.15, 0.2, 0.25, or 0.3 mm. In some embodiments, the sealed tube has a wall thickness of about 0.125 mm.

*Additive Manufacturing*

Additive manufacturing -colloquially referred to as 3D printing technology in the art- is one of the fastest growing applications for the fabrication of plastics. Components that make up the implant can be fabricated by additive techniques that allow for complex, non-symmetrical three-dimensional structures to be obtained using 3D printing devices and methods known to those skilled in the art (16, 17). There are currently three principal methods for additive manufacturing: stereolithography (SLA), selective laser sintering (SLS), and fused deposition modeling (FDM).

The SLA process requires a liquid plastic resin, a photopolymer, which is then cured by an ultraviolet (UV) laser. The SLA machine requires an excess amount of photopolymer to complete the print, and a common g-code format may be used to translate a CAD model into assembly instructions for the printer. An SLA machine typically stores the excess photopolymer in a tank below the print bed, and as the print process continues, the bed is lowered into the tank, curing consecutive layers along the way. Due to the smaller cross-sectional area of the laser, SLA is considered one of the slower additive fabrication methods, as small parts may take hours or even days to complete. Additionally, the material costs are relatively higher, due to the proprietary nature and limited availability of the photopolymers. In one embodiment, one or more components of the implant is fabricated by an SLA process.

The SLS process is similar to SLA, forming parts layer by layer through use of a high energy pulsed laser. In SLS, however, the process starts with a tank full of bulk material in powder form. As the print continues, the bed lowers itself for each new layer, advantageously supporting overhangs of upper layers with the excess bulk powder not used in forming the lower layers. To facilitate processing, the bulk material is typically heated to just under its transition temperature to allow for faster particle fusion and print moves, such as described in the art (*18*). In one embodiment, one or more components of the implant is fabricated by an SLS process.

Rather than using a laser to form polymers or sinter particles together, FDM works by extruding and laying down consecutive layers of materials at high temperature from polymer melts, allowing adjacent layers to cool and bond together before the next layer is deposited. In the most common FDM approach, fused fiber fabrication (FFF), polymer in the form of a filament is continuously fed into a heated print head print whereby it melts and is deposited onto the print surface. The print head moves in a horizontal plane to deposit polymer in a single layer, and either the print head or printing platform moves along the vertical axis to begin a new layer. A second FDM approach uses a print head design based on a traditional single-screw extruder to melt polymer granulate (powders, flakes, or pellets) and force the polymer melt through a nozzle whereby it is deposited on the print surface similar to FFF. This approach allows the use of standard polymer materials in their granulated form without the requirement of first fabricating filaments through a separate extrusion step. In one embodiment, one or more components of the implant is fabricated by an FDM and/or FFF process.

In another embodiment, Arburg Plastic Freeforming (APF) (19) is the additive manufacturing technique used in implant fabrication. In this embodiment, a plasticizing cylinder with a single screw is used to produce a homogeneous polymer melt similarly to the process for thermoplastic injection molding. The polymer melt is fed under pressure from the screw cylinder to a piezoelectrically actuated deposition nozzle. The nozzle discharges individual polymer droplets of controlled size in a pre-calculated position, building up each layer of the 3-dimensional polymer print from fused droplets. The screw and nozzle assembly is fixed in location, and the build platform holding the printed part is moved along three axes to control droplet deposition position. The droplets bond together on cooling to form a solid part. This technique can operate at elevated temperatures (ca. 300°C) and pressures (ca. 400 bar). One advantage of the APF method is that it is directly compatible with many of the processes used in injection molding and extrusion (e.g., granulated polymer feedstocks, no organic solvents).

In another embodiment, droplet deposition modelling (DDM) is used as the additive manufacturing technique by producing discrete streams of material during deposition, well-known in the art for inkjet systems.

A preferred method of additive manufacturing that avoids sequential layer deposition to form the three-dimensional structure is to use continuous liquid interface production (CLIP), a technique recently developed by Carbon3D. In CLIP, three dimensional objects are built from a fast, continuous flow of liquid resin that is continuously polymerized to form a monolithic structure with the desired geometry using UV light under controlled oxygen conditions. The CLIP process is capable of producing solid parts that are drawn out of the resin at rates of hundreds of mm per hour. Implant scaffolds containing complex geometries may be formed using CLIP from a variety of materials including polyurethane and silicone.

In one embodiment, the implants are manufactured under fully aseptic conditions. In another embodiment, the implants are terminally sterilized using methods known in the art such as gamma sterilization, steam sterilization, dry heat sterilization, UV irradiation, ethylene oxide sterilization, and the like.

**Methods for Implantation & Removal of the Device**

Examples of methods for insertion and removal of the device into the buccal cavity (or oral cavity) are described here. In some embodiments, one or more devices are implanted together. In one embodiment, insertion and removal are carried out by a dental practitioner, unlike other drug delivery implants known in the art (e.g., contraceptive subdermal implants) where a medical professional performs the procedures. This novel feature of the disclosed subject matter has the advantage of increasing accessibility to the device especially in remote, rural, or resource-limited regions.

*Insertion*

The devices of the present disclosure can be implanted into a subject/patient in accordance with standard procedures by trained professionals, who are experts in the art. The term "subject/patient" includes all mammals (e.g., humans, valuable domestic household, sport or farm animals, laboratory animals).

Advantageously, insertion (and removal) do not require aseptic techniques or preparation, an advantage of the targeted compartment sites (buccal vestibules, both mandibular and maxillary). Both insertion and removal are simple and rapid in an easily accessible area routinely visualized/palpated during any oral examination. Target site(s) are (optionally) treated with local anesthesia, followed by implant(s) insertion, positioning and rapid closure. There are no visible scars with low rate of morbidity while maintaining full form and function.

One or multiple implants are optionally provided to the healthcare professional performing the exam/insertion/-removal in a single kit, ready for use. The kit may also include gauze, forceps, scalpels and the like, all sterilized containers/kits (assuring sterile manufacturing and individual use).

In an embodiment, where more than one implant is planned for insertion, the second/others contain either the same or other pharmaceutical, microflora, nutritional, antigen/antibody or similar components (complimentary/unrelated but for distinct purposes). The second/others are inserted for a variety of reasons at a separate, delayed time point.

In another embodiment, insertion could instead by facilitated using a trocar to ease access and planar insertion along buccal vestibular walls. Such device insertion -and removal *(20,* 21)- are described in the art for other body compartments, for example subdermal implants, and are incorporated herein in full by reference (22, 23).

Once the targeted buccal vestibule (mandibular or maxillary, left or right) is identified and examined/ palpated and distal lingual nerve area defined insertion procedures can begin. Local anesthetic (often with slight vasoconstricting additives) is applied topically and/or injected at the mucosa site where the implant is planned for insertion. This site must be distal enough to accommodate the length of the implant. Once anaesthetized, a small horizontal mucosal incision (2-3 mm) is made, perpendicular to the mandible/maxillary bone along the preselected buccal vestibule. Initial blunt dissection using mosquito/similar forceps easily separates tissue planes, which will hold the implant in place. The implant is guided horizontally, in parallel alignment with the bony jaw. Depending on the flexibility of that particular implant, direct pushing will bluntly create the implant channel, enhanced by the specifically designed leading (proximal) edge. If the implant is of softer product profile, vestibular placement is easily assisted with gentle pressure on the external cheek to assist desired advancement.

In another embodiment, the insertion and guidance is facilitated using a pre-loaded trocar, adapted for these vestibules.

In another embodiment, two (or more) implants are inserted through the same initial insertion, although a second small incision (or extension of the first incision) is preferred in another embodiment to maintain distance between implants.

In another embodiment, these implants are positioned for malar and/or cheekbone defining aesthetics and/or repair (with no pharmaceutical agents, etc.) providing a space-filling purpose. In yet another embodiment, implants for reconstructive and/or plastics purposes include fibroblast and/or other similar agents to maintain tethering presence.

Once satisfactory insertion and positioning is achieved, optionally with ca. 5-7 mm distance between the insertion incision and the distal end of the implant to reduce likelihood of extrusion (entire procedure usually takes less than 1 minute), the forceps and pads are withdrawn. Based on professional's experience as well as confidence of insertion distance from incision, small resorbable sutures or similar agents are, in one embodiment, placed to ensure positioning, reduce extrusion risks and reduce any small possibility of bleeding.

Education is given to avoid biting down on implant for the first 12-36 hours.

*Removal*

Device removal notably does not require the practice of aseptic techniques. Removal strategies will vary depending on:

Anticipated (non-dissolvable or needing refill/replacement) or more emergent (inflammation and/or hypersensitivity reaction or other medically indicated reasons),

Technical repositioning,

Initial placement and number of implants (one only; one per buccal vestibule; more than one per buccal vestibule)

Patient request.

Dissolvable/resorbable implants are not anticipated to require removal under normal conditions.

Identification of targeted implant for removal is identified by palpation as well as use of imaging technique (ultrasound, magnetic detection, infrared, X-ray, or similar methods known in the art) based on the anticipated tracking markers incorporated into implant production.

Once identified, local anesthetic is applied topically/injected at the distal end of implant where small incision will be made (usually ca. 2-6 mm) enabling identifying the implant and/or retrieving adaptation (hole) using standard blunt-ended forceps or similar to visually identify the implant and grab/hook the distal end. Mosquito or similar forceps are then inserted to grab the end.

Based on implant components as well as individual responses, most are able to be grasped and pulled directly out without complications. Many are able to be "pushed out" with manual/instrument pressure on the back end/proximal implant end.

Occasionally, individuals may have more adherent submucosa, requiring lateral, blunt-ended, planar dissection alongside of implant for separation from fibrous tissue or other attaching elements. If two or more implants require removal at the same site, the incision may be made between implants, with blunt separation of layers to identify each implant; two often can be removed at the same time.

If indicated, one or more resorbable sutures or topical glue or similar methods are used to seal the incision although these will usually heal on their own. The afore mentioned advantages of the physiologically inflamed compartment (oral/buccal) for device placement reduces infection risk and complications.

*Implant Sheaths*

ePTFE has been used in the art as a sheath material to line the pocket where saline or silicone gel breast implants are surgically placed (*vide supra*). The ePTFE liners allow implants to integrate with the body by tissue in-growth without capsule (scar tissue) formation and also prevent the pocket from closing on itself, thus keeping the pocket open. A typical ePTFE liner is 0.35 mm thick and has a micro porosity of 40 $\mu$m. This allows the body to grow into pores without forming a fibrous scar around the material. The material is permanent and does not degrade within the body. It can be removed if necessary. In an embodiment, an ePTFE liner is placed in the buccal pocket where the implant(s) is(are) located, reducing the foreign body response and facilitating implant replacement for continuous therapies. The liner does not affect the drug release from the implant.

**Use and Applications of the Device**

A primary purpose of the buccal implant system described herein is to deliver one or more APIs to systemic circulation for the purposes of treating, preventing, reducing the likelihood of having, reducing the severity of and/or slowing the progression of a medical condition in a subject, also termed "application" hereunder. The primary purpose is augmented by the associated intent of increasing patient compliance by reducing problems in adherence to treatment and prevention associated with more frequent dosing regimens. Consequently, the disclosed invention can be used for a plurality of applications. Illustrative, non-restrictive examples of such applications are provided below in summary form. Based on these examples, one skilled in the art could adapt the disclosed technology to other applications.

Accordingly, provided are methods of treating or preventing a medical condition in a patient in need thereof, comprising implanting a sustained release drug delivery device into the oral mucosa of a subject, the device comprising a matrix, reservoir, or hybrid design comprising one or more thermoplastic polymers, elastomer materials, or metals suitable for pharmaceutical use and a therapeutically effective amount of one or more active pharmaceutical ingredients. In some embodiments, implanting the sustained release drug delivery device into the oral mucosa is not performed with aseptic techniques or preparation.

In some embodiments, the medical condition is an infectious disease, a transplant graft rejection, a condition indicating hormonal therapy, a physiological or pathophysiological application, diabetes mellitus, an allergy or hypersensitivity, an autoimmune disorder, cancer, a hematological disease, a musculoskeletal disorder, a psychological or neurological disorder, a genetic disease, or a veterinary condition. Nonlimiting examples of medical conditions or circumstances wherein delivery of an active agent (e.g., sustained, long term release/administration of an active agent) is desired are presented herein below.

*Infectious Diseases,* including multiple, overlapping infections:

HIV prevention using one or more one or more suitable antiretroviral agents, including biologics, and/or one or more vaccines and/or adjuvants delivered from the implant; and treatment, using one or more suitable antiretroviral agents, including biologics, delivered from the implant,

Sexually transmitted infections (STIs), including but not limited to prevention or treatment, both active and chronic active, with one or more suitable antimicrobial agents delivered from the implant. Illustrative, but not limiting examples of STIs include: gonorrhea, chlamydia, lymphogranuloma venereum, syphilis, including multidrug-resistant (MDR) organisms, hepatitis C virus, and herpes simplex virus,

Hepatitis B virus (HBV) prevention or treatment, both active and chronic active, with one or more suitable antiviral agents delivered from the implant,

Herpes simplex virus (HSV) and varicella-zoster virus (shingles) Zoster/Shingles, prevention or treatment, both active and chronic active, with one or more suitable antiviral agents delivered from the implant,

Cytomegalovirus (CMV) and congenital CMV infection, prevention or treatment, both active and chronic active, with one or more suitable antiviral agents delivered from the implant,

Malaria, prevention or treatment, both active and chronic active, with one or more suitable antimicrobial agents delivered from the implant,

Tuberculosis, including multidrug-resistant (MDR) and extensively drug-resistant (XDR) tuberculosis, prevention or treatment, both active and chronic active, with one or more suitable antibacterial agents delivered from the implant,

Acne, treatment or management with one or more suitable agents delivered from the implant.

Respiratory viral infections including, but not limited to influenza viruses and coronaviruses, for example SARS-CoV-2.

Influenza spreads around the world in seasonal epidemics, resulting in the deaths of hundreds of thousands annually-millions in pandemic years. For example, three influenza pandemics occurred in the 20th century and killed tens of millions of people, with each of these pandemics being caused by the appearance of a new strain of the virus in humans. Often, these new strains result from the spread of an existing influenza virus to humans from other animal species. Influenza viruses are RNA viruses of the family Orthomyxoviridae, which comprises five genera: Influenza virus A, Influenza virus B, Influenza virus C, Isavirus and Thogoto virus. The influenza A virus can be subdivided into different serotypes based on the antibody response to these viruses. The serotypes that have been confirmed in humans, ordered by the number of known human pandemic deaths, are: H1N1 (which caused Spanish influenza in 1918), H2N2 (which caused Asian Influenza in 1957), H3N2 (which caused Hong Kong Flu in 1968), H5N1 (a pandemic threat in the 2007-08 influenza season), H7N7 (which has unusual zoonotic potential), H1N2 (endemic in humans and pigs), H9N2, H7N2, H7N3 and H10N7. Influenza B causes seasonal flu and influenza C causes local epidemics, and both influenza B and C are less common than influenza A.

Coronaviruses are a family of common viruses that cause a range of illnesses in humans from the common cold to severe acute respiratory syndrome (SARS). Coronaviruses can also cause a number of diseases in animals. Coronaviruses are enveloped, positive-stranded RNA viruses whose name derives from their characteristic crown-like appearance in electron micrographs. Coronaviruses are classified as a family within the Nidovirales order, viruses that replicate using a nested set of mRNAs. The coronavirus subfamily is further classified into four genera: alpha, beta, gamma, and delta coronaviruses. The human coronaviruses (HCoVs) are in two of these genera: alpha coronaviruses (including HCoV-229E and HCoV-NL63) and beta coronaviruses (including HCoV-HKU1, HCoV-OC43, Middle East respiratory syndrome coronavirus (MERS-CoV), the severe acute respiratory syndrome coronavirus (SARS-CoV), and SARS-CoV-2).

*Transplants - Graft Rejection:*

Chronic immune-suppressive post-transplant therapy with one or more suitable agents delivered from the implant.

*Hormonal Therapy:*

Contraception, including estrogens and progestins, with one or more suitable agents delivered from the implant,

Hormone replacement, with one or more suitable agents delivered from the implant,

Testosterone replacement, with one or more suitable agents delivered from the implant,

Thyroid replacement/blockers, with one or more suitable agents delivered from the implant,

Hormonal treatment to regulate triglycerides (TGs) using one or more suitable agents delivered from the implant,

Chronic pharmacologic support for all transgender individuals (all stages from cis-trans), using one or more suitable agents delivered from the implant.

*Physiology and Pathophysiology:*

Gastrointestinal (GI) applications, with one or more suitable agents delivered from the implant, including, but not limited to the treatment/management of diarrhea, pancreatic insufficiency, cirrhosis, fibrosis in all organs; GI organs-related parasitic diseases, gastroesophageal reflux disease (GERD),

Cardiovascular applications, with one or more suitable agents delivered from the implant, including, but not limited to the treatment/management of hypertension (HTN) using, for example, statins or equivalent, cerebral/peripheral vascular disease, stroke/emboli/arrhythmias/deep venous thrombosis (DVT) using, for example anticoagulants and anti-atherosclerotic cardiovascular disease (ASCVD) medications, and congestive heart failure (CHF) using for example β-blockers, ACE inhibitors, and angiotensin receptor blockers,

Pulmonary applications, with one or more suitable agents delivered from the implant, including, but not limited to the treatment/management of sleep apnea, asthma, longer-term pneumonia treatment, pulmonary HTN, fibrosis, and pneumonitis,

Bone applications, with one or more suitable agents delivered from the implant, including, but not limited to the treatment/management of chronic pain (joints as well as bone including sternal), osteomyelitis, osteopenia, cancer, idiopathic chronic pain, and gout,

Urology applications, with one or more suitable agents delivered from the implant, including, but not limited to the treatment/management of benign prostatic hyperplasia (BPH), bladder cancer, chronic infection (entire urologic system), chronic cystitis, prostatitis,

Ophthalmology applications, with one or more suitable agents delivered from the implant, including, but not limited to the treatment/management of glaucoma, ocular infections,

Cholesterol management, with one or more suitable agents delivered from the implant,

Metabolic applications, with one or more suitable agents delivered from the implant, including, but not limited to the treatment/management of weight gain, weight loss, obesity, malnutrition (replacement), osteopenia, Vitamin deficiency (B vitamins/D), folate, and smoking/drug reduction/cessation.

*Diabetes mellitus*:

Treatment and management of diabetes (type 1 and 2), with one or more suitable agents (including peptide drugs) delivered from the implant,

*Allergies and Hypersensitivities,* with "desensitization", often need low-dose repeated exposure:

TYPES: Type I (IgE mediated reactions), Type II (antibody mediated cytotoxicity reactions), Type III (immune complex-mediated reactions), and Type IV for delayed type hypersensitivity (24), with one or more suitable agents delivered from the implant,

Hypersensitivity reactions (HSRs), with one or more suitable agents delivered from the implant,

Antibiotics, biologics (drug and antibody portion), chemotherapy (e.g., platins), progesterone, as well as other treatments known in the art and described in (24), with one or more suitable agents delivered from the implant,

Food allergies (e.g., nuts, shellfish) with one or more suitable agents delivered from the implant,

Allergy medication dosing with one or more suitable agents delivered from the implant, as an alternative to allergy shots, recommended for people with severe allergy symptoms who do not respond to usual medications; for people who have significant medication side effects from their medications; for people who find their lives disrupted by allergies/insect stings; or people for whom allergies might become life threatening: anaphylaxis.

*Autoimmune Disorders,* often classified as chronic inflammatory disorders:

Treatment and management of Crohn's disease and ulcerative colitis, with one or more suitable agents (e.g., biologics) delivered from the implant,

Rheumatoid arthritis (RA) treatment and management with one or more suitable agents (e.g., biologics) delivered from the implant,

Multiple sclerosis (MS) treatment and management with one or more suitable agents (e.g., biologics) delivered from the implant,

Psoriasis treatment and management with one or more suitable agents (e.g., biologics) delivered from the implant,

Lupus treatment and management with one or more suitable agents (e.g., biologics) delivered from the implant,

Autoimmune thyroiditis treatment and management with one or more suitable agents (e.g., biologics) delivered from the implant.

*Oncology:*

Chemotherapy and targeted therapy (e.g., Ig) chronic or sub-chronic cancer management with one or more suitable agents delivered from the implant.

*Hematologic Diseases:*

Treatment/management of Hemophilia A with one or more suitable agents (e.g., Factor VIII orthologs) delivered from the implant,

Administration of anticoagulants and/or antiplatelet therapy with one or more suitable agents delivered from the implant,

Treatment/management of leukemia/lymphoma and bone marrow transplant (MBT) therapies with one or more suitable agents delivered from the implant,

Iron replacement therapy with one or more suitable agents delivered from the implant,

Fibroproliferative disorders required blockade.

*Musculoskeletal Applications:*

Delivery of one or more anti-inflammatory agents (e.g., NSAIDS) from the implant,

Delivery of low-dose prednisone from the implant,

Opioids addiction/pain management with one or more suitable agents delivered from the implant,

Hypertrophic fibrosis/scar tissue.

*Psychological and Neurologic Disorders:*

Treatment and management of depression with one or more suitable agents delivered from the implant,

Treatment and management of schizophrenia, and related, with one or more suitable agents delivered from the implant,

Treatment and management of bipolar disorders with one or more suitable agents delivered from the implant,

Treatment and management of dysthymic disorders with one or more suitable agents delivered from the implant,

Treatment and management of seizure control with one or more suitable agents delivered from the implant,

Treatment and management of ADD/ADHD and hyperactivity disorders with one or more suitable agents delivered from the implant,

Treatment and management of behavioral/emotional secondary to early-onset (child/adolescent), substance use, physical, sexual, emotional abuse, PTSD, and anxiety with one or more suitable agents delivered from the implant,

Treatment and management of Parkinson's disease with one or more suitable agents delivered from the implant,

Treatment and management of Alzheimer's disease with one or more suitable agents delivered from the implant.

*Genetic Diseases:*

Treatment of congenital genetic deficiency diseases, including genetic excess diseases, with one or more suitable agents delivered from the implant,

Treatment of primary immunodeficiencies (e.g., agammaglobulinemia, secretory IgA deficiency, sIgA deficiency) with one or more suitable agents delivered from the implant,

Severe combined immunodeficiency (SCID) treated SCID with one or more suitable agents delivered from the implant, including, but not limited to enzyme replacement therapy (ERT) with pegylated bovine ADA (PEG-ADA),

Muscular dystrophy treated and managed with one or more suitable agents delivered from the implant,

Treatment or management of Duchenne's disease with one or more suitable agents (e.g., eteplirsen) delivered from the implant,

Treatment or management of Pompe's disease with one or more suitable agents delivered from the implant, including ERT such as intravenous administration of recombinant human acid $\alpha$-glucosidase,

Treatment or management of Gaucher disease with one or more suitable agents delivered from the implant, including ERT.

*Veterinary Applications* involving all mammals, including, but not limited to dogs, cats, horses, pigs, sheep, goats, and cows.

In one embodiment, the buccal implant serves multiple purposes, where more than one application is targeted simultaneously. An example of such a multipurpose buccal implant involves the prevention of HIV infection, with the delivery of one or more antiretroviral agents, and contraception, with the delivery of one or more contraceptive agents. In some embodiments, provided are methods of treating HIV.

In one embodiment, the buccal implant serves multiple purposes, where one or more drug delivery applications are targeted in addition to cosmetic and/or sculptural function performed by the device at the implantation site. The post-treatment application of the buccal implant -for example, but not limited to, following: surgery, chemotherapy, radiation, motor-vehicle accidents (MVAs), war-related injuries- can included embodments where the device is used as a filler for both the internal oral cavity and the external facial areas, especially if coated with fibroblast or other stimulating factors. These embodments make up a novel combination of uses. These embodments are also contemplated under "treatment or prevention of a medical condition."

In some embodiments, provided are methods of treating or preventing a medical condition in a patient in need thereof comprising implanting a sustained release drug delivery device into the oral mucosa of a subject, the device comprising a matrix, reservoir, or hybrid design comprising one or more thermoplastic polymers, elastomer materials, or metals suitable for pharmaceutical use and a therapeutically effective amount of one or more active pharmaceutical ingredients. In some embodiments, the one or more active pharmaceutical ingredients are selected from antiretrovirals, antimicrobial agents, antibacterial agents, antivirals, hormones, statins, $\beta$-blockers, ACE inhibitors, angiotensin receptor blockers, vitamins, steroids, biologics, anti-cancer drugs, allergy medications, anticoagulants, antiplatelet therapies, non-steroidal anti-inflammatory drugs, vaccines, and combinations thereof. In some embodiments, the one or more active pharmaceutical ingredients comprise atazanavir, didanosine, efavirenz, emtricitabine, lamivudine, lopinavir, nevirapine, raltegravir, ritonavir, saquinavir, stavudine, tenofovir, tenofovir disoproxil fumarate, tenofovir alafenamide, zidovudine, acyclovir, famciclovir, valcyclovir, morphine, buprenorphine, estrogen, progestin, progesterone, cyclosporine, a calcineurin inhibitor, prostaglandin, cabotegravir, rilpivirine, lenacapavir (GS-6207), 4'-ethynyl-2-fluoro-2'-deoxyadenosine (MK-8591, EFdA), a beta-blocker, gentamycin, corticosteroid, a fluoroquinolone, insulin, an antineoplastic drug, anti-nausea drug, a corticosteroid, an antibiotic, morphine buprenorphine, a VEGF inhibitor, or combinations thereof. In some embodiments, the one or more active pharmaceutical ingredients comprise tenofovir. In some embodiments, the one or more active pharmaceutical ingredients comprise tenofovir alafenamide.

In some embodiments, provided are methods of delivering an active pharmaceutical ingredient to a subject. In some embodiments, provided are methods of delivering an active pharmaceutical ingredient to a subject, comprising implanting a sustained release drug delivery device into the oral mucosa of a subject, the device comprising a matrix, reservoir, or hybrid design comprising one or more thermoplastic polymers, elastomer materials, or metals suitable for pharmaceutical use and a therapeutically effective amount of one or more active pharmaceutical ingredients. In some embodiments, the one or more active pharmaceutical ingredients are selected from antiretrovirals, antimicrobial agents, antibacterial agents, antivirals, hormones, statins, $\beta$-blockers, ACE inhibitors, angiotensin receptor blockers, vitamins, steroids, biologics, anti-cancer drugs, allergy medications, anticoagulants, antiplatelet therapies, non-steroidal anti-inflammatory drugs, vaccines, and combinations thereof. In some embodiments, the one or more active pharmaceutical ingredients comprise atazanavir, didanosine, efavirenz, emtricitabine, lamivudine, lopinavir, nevirapine, raltegravir, ritonavir, saquinavir, stavudine, tenofovir, tenofovir disoproxil fumarate, tenofovir alafenamide, zidovudine, acyclovir, famciclovir, valcyclovir, morphine, buprenorphine, estrogen, progestin, progesterone, cyclosporine, a calcineurin inhibitor, prostaglandin, cabotegravir, rilpivirine, lenacapavir (GS-6207), 4'-ethynyl-2-fluoro-2'-deoxyadenosine (MK-8591, EFdA), a beta-blocker, gentamycin, corticosteroid, a fluoroquinolone, insulin, an antineoplastic drug, anti-nausea drug, a corticosteroid, an antibiotic, morphine buprenorphine, a VEGF inhibitor, or combinations thereof. In some embodiments, the one or more active pharmaceutical ingredients comprise tenofovir. In some embodiments, the one or more active pharmaceutical ingredients comprise tenofovir alafenamide.

## OTHER CONSIDERATIONS

The present disclosure is novel and not obvious to one skilled in the art, as discussed above and in the ensuing section. The disclosed invention is one or more drug delivery devices to be implanted in the buccal compartment **(FIG 1),**

located in the oral cavity. The device(s) delivers one or more APIs to the systemic compartment in a controlled, sustained fashion for a duration of use of five days, or longer.

While the sustained delivery of APIs in the oral cavity has been explored, the technologies are only =fundamentally different from the invention disclosed herein and/or have been applied in totally different compartments in the oral cavity (i.e., the gingiva) for periodontal, not systemic, indications.

There is at least one non-obvious aspect of the disclosed invention that goes completely against accepted dogma in the art. The oral cavity, and the buccal compartment in particular, is microbe-rich and diverse body habitat identified in the Human Microbiome Project as one of the five key anatomic niches where the microbial taxa are key contributors to human health and disease (4). Sterile insertion methods for implants is the *sine qua non* in practice with, aseptic techniques held as the "standard of care". It is, therefore, non-obvious to one expert in the art to inject or implant a drug delivery device, especially for chronic use, without using aseptic techniques, in microbe-rich and physiologically inflamed buccal tissue. In fact, such practice would go against recommendations from the Centers for Disease Control and Prevention (*30*) and the World Health Organization (31). The latter states that, "an aseptic technique should be followed for all injections" and to "avoid giving injections if your skin integrity is compromised by local infection or other skin conditions". The buccal surface cannot be disinfected as prescribed in the World Health Organization guidelines by applying "a 60-70% alcohol-based solution (isopropyl alcohol or ethanol) on a single-use swab of cotton-wool ball" *(31)*.

It is commonly accepted in the art that sterile techniques are essential for injecting medications. Skin and needle hygiene have been studied in the context of injection drug use (IDU) and it was found that non-sterile techniques can result in bacterial infections and that "these infections cause significant morbidity" and "can develop into life-threatening infections that can lead to hospitalization requiring extensive IV antibiotics" (32). Phillips *et al.* further state that, "microbiologic research has established that an injector's skin flora may be the most important source leading to bacterial infections among IDUs" (32).

Based on the above established practices, it is not surprising that devices with the purpose of delivering drugs systemically for extended periods of time are implanted aseptically subdermally -for example, contraceptive implants such as the Implanon (2)- and not buccally. The skin can be disinfected easily (see above) and hosts much lower abundances of bacterial taxa than the oral cavity, especially the buccal environment.

The discreteness of the implanted devices is another non-obvious, novel feature of the invention disclosed herein. In many of the embodiments described above the implant, or multiple implants, are not visible or palpable from the exterior of the oral cavity. This is an advantageous aspect of the disclosed invention as it is often desirable for a subject or patient to keep the use of the implant private. For example, there is considerable stigmatization in sub-Saharan Africa for women who wish to use a prophylactic regimen for the prevention of HIV infection.

Finally, because the anatomic compartment specified here for implant use consists uniquely of the buccal mucosa, the devices can be implanted and removed by dental practitioners rather than medical professionals. This is another novel aspect of the disclosed invention and makes it to be more accessible in resource-limited regions.

## EQUIVALENTS

The present disclosure is not to be limited in terms of the particular embodiments described in this application, which are intended as illustrations of various aspects. Many modifications and variations can be made without departing from the spirit and scope of the disclosure, as will be apparent to those skilled in the art. Functionally equivalent methods, systems, and apparatus within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds compositions or biological systems, which can, of course, vary. It is also to be understood that the terminology used herein is for the purpose of describing particular embodiments only, and is not intended to be limiting. As will be understood by one skilled in the art, for any and all purposes, such as in terms of providing a written description, all ranges disclosed herein also encompass any and all possible subranges and combinations of subranges thereof.

As a person skilled in the art would readily know many changes can be made to the preferred embodiments without departing from the scope thereof. It is intended that all matter contained herein be considered illustrative and not in a limiting sense.

While various aspects and embodiments have been disclosed herein, other aspects and embodiments will be apparent to those skilled in the art.

## ADDITIONAL EMBODIMENTS

Additional embodiments of the disclosure have one or more of the aspects defined below.

In some cases, the buccal implants comprise one or more of the following:

- One or more compartments that contain one or more APIs and make up a significant portion of the device volume, also known as "kernels".
- One or more skin layers permeable to the API(s) covering one or more kernels and meeting one or more of the following requirements:

    a) Act as diffusion-limiting barriers to control the release of the APIs from the central compartment,
    b) Protect the central compartment from one or more components of the external environment, and/or
    c) Provide structural support to the device.

    The skin optionally consists of a continuous membrane that covers all or part of the device. It is not perforated with orifices or channels that are generated during device fabrication (e.g., mechanical punching, laser drilling).
- Defined microscopic pore structure that is distributed throughout a solid matrix. The pore structure is incorporated into one, or both, of the above elements. In other words, one or more kernels and/or one or more skins have a microscopic pore structure. A "microscopic pore" structure is defined as known by those skilled in the art as follows:

    a) Microporous, with defined pores that have diameters smaller than 2 nm,
    b) Mesoporous, with defined pores that have diameters between 2 - 50 nm.

- Macroporous, with defined pores that have diameters larger than 50 nm and typically smaller than 50 $\mu$m. "Skin" is defined by a low volume element of the drug delivery system that covers part or all of a kernel.

"Skin" means the outer portion of the drug delivery system that contacts the external environment. The terms "skin", "membrane", and "layer" are used herein interchangeably.

"Rate limiting skin" is a specific embodiment of a skin defined by the part of the system which comprises of polymer(s) with relatively low permeability for the therapeutic agents.

"Permeability" means the measurement of a therapeutic agent's ability to pass through a thermoplastic polymer.

The devices embodying these foundational elements contain a hierarchical structure based on three levels of organization:

**Primary structure:** Based on the physicochemical properties of the components and materials that make up the kernel and skin of the implant. This includes, but is not limited to, elements such as polymer or elastomer composition, molecular weight, crosslinking extent, hydrophobicity/hydrophilicity, and rheological properties; and/or drug physicochemical properties such as solubility, log P, and potency.

**Secondary structure:** The complex microstructure of the kernel and/or the skin. This can include, but is not limited to, properties such as the drug particle size, shape, and structure (e.g., core-shell architecture); fiber structures of drug or excipients in kernel; and/or pore properties (pore density, pore size, pore shape, etc.) of sponge-based kernel materials or of porous skins.

**Tertiary structure:** The macroscopic geometry and architecture of the implantable device. This includes elements such as, but not limited to, implant size and shape; kernel and skin dimensions (thickness, diameter, etc.); and/or layers of kernel and/or skin and their relative orientation.

Incorporation of these elements in an implantable drug-delivery device determines the characteristics of controlled, sustained delivery of one or more APIs at a predetermined location in the body (i.e., the implantation site).

The device as described herein is intended to be left in place for periods of time spanning one day to one year, or longer, and delivers one or more APIs during this period of use. In certain exemplary, nonlimiting embodiments, the devices are implanted in the buccal cavity (or oral cavity) and deliver one or more APIs for 3-12 months.

Implant geometries are based on multiple shapes. In one exemplary, nonlimiting embodiment, the shape of the device is based on a cylinder, and in some cases, the ends of the cylinder are joined to afford a toroid. These geometries are well-known in the art.

In some embodiments, the geometry may be a rectangular prism. Cylindrical or rectangular prism geometries may be flat, or may have a curved shape. In another embodiment, the implant is shaped like a capsule, from about 3 to about 50 mm in diameter and up to about 5 mm in height.

In another embodiment, the powder making up the reservoir kernel consists of microscale (1 - 1,000 $\mu$m cross-section) or nanoscale (1 - 1,000 nm cross-section) drug carriers. The drug carriers are particulate materials containing the API, either internally or on the surface. Nonlimiting examples of such carriers, known in the art, are beads; capsules; microgels, including but not limited to chitosan microgels; nanocelluloses; dendrimers; and diatoms. The carriers are filled or coated with API using impregnation or other methods known in the art (e.g., lyophilization, rotary solvent evaporation, spray-drying).

*Fiber-based Systems*

In another embodiment, the drug kernel may consist of drug dispersions in high surface area fiber-based carriers, known in the art in tissue engineering, delivery of chemotherapeutic agents, and wound management devices, as described in. In one embodiment, the high surface area carrier consists of fibers produced by electrospraying. In one embodiment, the high surface area carrier consists of electrospun fibers, including, but not limited to electrospun nanofibers. The use of electrospun fibers in drug delivery is well-known.

Electrospun, drug-containing fibers can have a number of configurations well-known in the art. For example, in one embodiment, the API is embedded in the fiber, a miniaturized version of the above matrix system. In another exemplary embodiment, the API-fiber system is produced by coaxial electrospinning to give a core-shell structure, a miniaturized version of the above reservoir system. Core-shell fibers production by coaxial electrospinning is known in the art to produce encapsulation of water-soluble agents, such as biomolecules including, but not limited to proteins, peptides, and the like. In yet another exemplary embodiment, Janus nanofibers can be prepared by methods known in the art. Janus fibers contain two or more separate surfaces having distinct physical or chemical properties, the simplest case being two fibers joined along an edge coaxially.

At least part of the fiber-based devices disclosed herein are covered with one or more skins, as described more fully under "The Implant Skin".

Electrospun fibers may be used to form the kernel of a reservoir implant. In one embodiment, a reservoir implant is formed by packing drug-containing fibers into a tubular implant skin and sealing the tube ends ad described in a subsequent section. Fibers formed by electrospinning may be collected on a plate or other flat surface and chopped, ground, or otherwise reduced in size by methods known in the art to a size that can be effectively packed into the implant, forming a packed powder kernel. The resulting reduced-sized electrospun fiber material may also be formulated into a kernel using any of the methods described herein for drug powder or drug-excipient powder mixtures. In an alternative embodiment, the electrospun fibers may be collected on a fixed or stationary collector surface (e.g., a plate or drum) in the form of a mat. The mat may be subsequently cut to an appropriate size and geometry (e.g., cut into strips or sheets), and placed in a tubular skin structure to form a reservoir implant. In another embodiment, the electrospun, drug-containing mat may be rolled into a multi-layer cylindrical shape to form the kernel of a tubular reservoir implant. In yet another embodiment, the kernel is formed from an electrospun fiber yarn fabricated using methods known in the art. In another embodiment, an electrospun fiber kernel in a cylindrical geometry may be prepared by collecting fibers during the spinning process directly on a rotating wire, fiber, or small diameter mandrel.

Electrospinning may also be used to create skins. In one embodiment, a membrane or mat of electrospun fibers collected on a rotating plate or drum may be used as a skin. Skins formed in this fashion may be wrapped around a preformed kernel to form a reservoir implant, or may be rolled into a tubular shape and be filled with a kernel material and sealed. Alternatively, a tubular skin may be formed directly by collecting electrospun fibers on a rotating mandrel during the spinning process.

An alternative embodiment utilizes electrospinning processes to fabricate both the kernel and skin, using the methods described herein for each. In yet another embodiment, electrospinning may be used to form the skin layer, kernel layer, or both in layered implant embodiments described in a subsequent section.

*Porous Sponge Systems*

In some embodiments, the implant kernel consists of a porous support structure containing the drug. The support has a porous microstructure (pore sizes 1-1,000 $\mu$m). In some embodiments, the support has a porous nanostructure (pore sizes 1-1,000 nm). In yet other embodiments, the support has both porous microstructures and nanostructure. Examples of these microscopic pores include, but are not limited to sponges, including: silica sol-gel materials; xerogels; mesoporous silicas; polymeric microsponges; including polydimethylsiloxane (PMDS) sponges and polyurethane foams; nanosponges, including crosslinked cyclodextrins; and electrospun nanofiber sponges and aerogels.

In one embodiment, the implant kernel consists of sponge structure known in the art - illustrative examples are provided above- and the drug is incorporated by impregnation using methods known in the art. In some embodiments, the sponges are magnetic to enable, for example, remotely triggered drug release. This method is known in the art.

In one embodiment, the sponge pores are created *in situ* during use using a templating excipient. A number or porogens are known in the art and have been used to generate porous structures. Methods for creating pores during use (i.e., *in vivo*) include, but are not limited to, the inclusion of excipient particles in implant core that dissolve when exposed to bodily fluids, such as subcutaneous fluid. As used herein, solid particles can include crystalline or amorphous forms. In one embodiment, the size distribution of the solid particles is polydisperse. In one embodiment, the size distribution of the solid particles is monodisperse. In one embodiment, the solid particles consist of nanoparticles (mean diameter < 100 nm). In one embodiment, the mean diameter of the particles can range from 1 - 10 nm, 10 - 25 nm, 25 - 100 nm, and 100 - 500 nm. Suitable mean microparticle diameters can range from 0.5 - 50 $\mu$m, from 0.5 - 5 $\mu$m, from 5 - 50 $\mu$m, from 1 - 10 $\mu$m, from 10 - 20 $\mu$m, from 20 - 30 $\mu$m, from 30 - 40 $\mu$m and from 40 - 50 $\mu$m. Other suitable mean particle diameters can range from 50 - 500 $\mu$m, from 50 - 100 $\mu$m, from 100 - 200 $\mu$m, from 200 - 300 $\mu$m, from 300 - 400 $\mu$m, from 400 - 500 $\mu$m, and from 0.5 - 5 mm. Suitable particle shapes include spheres, needles, rhomboids, cubes, and irregular shapes. Said templating particles can consist of salts (e.g., sodium chloride), sugars (e.g., glucose), or other water-soluble excipients known in the art. One skilled in the art would know how to produce such particles of well-defined shape and size. The mass ratio of pore-forming particles to API in the core ranges from 100 to 0.01. More specifically, said ratio can range from 100 - 20, from 20 - 5, or from 5 - 1. In other embodiments, the ratio can range from 1 - 0.2, from 0.2 - 0.05, or from 0.05 - 0.01.

In some embodiments, the pores are formed during manufacture (i.e., prior to use) by immersing the device in a suitable fluid (e.g., water or organic solvent) to dissolve the porogens.

In some embodiments, the pores can form as a result of mechanical, temperature, or pH changes following implantation/use.

*The Implant Skin*

It is advantageous to have a skin as part of the disclosed devices, which can cover the kernel partially or in its entirety.

The *in vitro* and *in vivo* drug release profile of the matrix implants disclosed herein generally are non-linear, with an initial burst of drug release followed by a low, sustained release phase. In certain indications, it may be desirable to linearize the drug release properties of the implant. In an advantageous embodiment of such a buccal implant, the external surface of the device is covered by a rate-limiting skin. In one embodiment, the skin is made up of a biocompatible elastomer. The composition and thickness of the skin determines the extent of linearization of the drug release as well as the rate of drug release. The skin thickness can range from 5 - 700 $\mu$m. Suitable thicknesses of the skin can range from 5 - 700 $\mu$m, from 10 - 500 $\mu$m, from 15 - 450 $\mu$m, from 20 - 450 $\mu$m, from 30 - 400 $\mu$m, from 35 - 350 $\mu$m, and from 40 - 300 $\mu$m. In certain embodiments the thickness of the skin is 10 $\mu$m, 20 $\mu$m, 30 $\mu$m, 40 $\mu$m, 50 $\mu$m, 60 $\mu$m, 70 $\mu$m, 80 $\mu$m, 90 $\mu$m, 100 $\mu$m, 125 $\mu$m, 150 $\mu$m, 175 $\mu$m, 200 $\mu$m, 225 $\mu$m, 250 $\mu$m, and 300 $\mu$m. In some embodiments, the thickness of the skin is 30 $\mu$m, 50 $\mu$m or 80 $\mu$m. These skin characteristics also apply to reservoir-type designs.

In one series of embodiments, a single external skin encases the API-containing compartment. In another embodiment a plurality of external skins encases the API-containing compartment. In some embodiments 2 - 20 independent, layered skins encase the API-containing compartment. In some embodiments, these skins or membranes consist of the same material, with the same or different thicknesses. In some embodiments, these skins or membranes consist of one or more different materials, with the same or different thicknesses.

In another series of embodiments, a plurality of skins is distributed throughout the device isolating different regions of the main component volume from each other. In one embodiment, one can envision these interspersed skins by analogy to the rings in a tree trunk. The skins in such embodiments can consist of one or more different materials, with the same or different thicknesses. The volumes (kernels) separated by the skins can all contain the same API at the same concentration, or different APIs at different concentrations. Some of said volumes may be unmedicated. Excipients in or making up said volumes can be the same or different across compartments separated by the skins.

In certain embodiments described herein, the implant kernel can be a single compartment. In other embodiments, the kernel of the drug delivery systems described herein may be comprised of two compartments in a segmented arrangement or arranged in two layers. In other embodiments, the kernel of the drug delivery systems described herein may be comprised of more than two compartments or layers. Each kernel may contain one or more therapeutic agents, or no therapeutic agents. For example, in certain embodiments of the buccal implant drug delivery systems described herein, the kernel is comprised of a first layer and a second layer, wherein the second layer is adjacent to the skin, and the first layer is adjacent to the second layer. A second skin layer may optionally be present adjacent to the first skin layer. In certain embodiments, one or more skin layers may contain a therapeutic agent as described previously. In one embodiment, the first kernel is completely surrounded by the second kernel. Only the second kernel is in contact with the first skin layer. In an alternate embodiment, the first kernel is concentric with the second kernel but a portion of the first kernel contacts the first skin layer at the implant end. The first skin layer may be continuous around the entire implant, or it may be composed of a second material in the form of an end cap that contacts the first kernel. In a further embodiment, the first kernel is separated from the second kernel by a barrier layer, that does not contain a therapeutic agent. Optional first and second skin layers may be present adjacent to the second kernel.

In certain embodiments, the first, second and third layers of the kernel are made from the same polymer. However, it can be envisioned that different polymers can be used for the first, second and third layers of the core so long as the first therapeutic agent in the kernel experiences a reduced permeation resistance as it is being released through the skin and meets the necessary release criteria needed to achieve a desired therapeutic effect.

In yet another series of embodiments, one or more skins can be medicated with one or more APIs. In certain embodiments, the first therapeutic agent is in dissolved form in the kernel and the second therapeutic agent is in solid form in the skin. As used herein, the term "solid form" can include crystalline or amorphous forms. In certain embodiments, the first therapeutic agent is in solid form in the and the second therapeutic agent is in solid form in the skin. In certain embodiments, the first therapeutic agent is in solid form in the kernel and the second therapeutic agent is in dissolved form in the skin. In certain embodiments, the first therapeutic agent is in the kernel of a reservoir-type system and the second therapeutic agent is in solid form in the skin. As used herein, the term "solid form" can include crystalline or amorphous forms. In certain embodiments, the first therapeutic agent is in the kernel of a reservoir-type system and the second therapeutic agent is in dissolved form in the skin.

In one embodiment, the skin is non-resorbable. It may be formed of a medical grade silicone, as known in the art. Other examples of suitable non-resorbable materials include synthetic polymers selected from poly(ethers), poly(acrylates), poly(methacrylates), poly(vinyl pyrolidones), poly(vinyl acetates), including, but not limited to poly(ethylene-co-vinyl acetate), or ethylene vinyl acetate (EVA), poly(urethanes), celluloses, cellulose acetates, poly(siloxanes), poly(ethylene), poly(tetrafluoroethylene) and other fluorinated polymers, poly(siloxanes), copolymers thereof, and combinations thereof. The implant shell may also consist of a biocompatible metal such as titanium, stainless steel, and others known in the art.

In one embodiment, one or more skins consist of the non-resorbable polymer expanded poly(tetrafluoroethylene) (ePTFE), also known in the art as Gore-Tex.

In some embodiments, the buccal implant device comprises expanded polytetrafluoroethylene (ePTFE) having microscopic pores. In some embodiments, the ePTFE has a density of about 0.25 to about 1.5 g cm$^{-3}$, e.g., about 0.25, 0.3, 0.35, 0.4, 0.45, 0.5, 0.55, 0.6, 0.65, 0.7, 0.75, 0.8, 0.85, 0.9, 0.95, 1.0, 1.05, 1.1, 1.15, 1.2, 1.25, 1.3, 1.35, 1.4, 1.45, or 1.5 g cm$^{-3}$. In some embodiments, the ePTFE has a density of about 0.35, 0.5, 0.85, or 1.15 g cm$^{-3}$.

In some embodiments, the buccal implant device comprises a rate-limiting skin. In some embodiments, the rate-limiting skin comprises expanded polytetrafluoroethylene (ePTFE).

In another embodiment, the implant shell is resorbable. In one embodiment of a resorbable device, the sheath is formed of a biodegradable or bioerodible polymer. Examples of suitable resorbable materials include synthetic polymers selected from poly(amides), poly(esters), poly(ester amides), poly(anhydrides), poly(orthoesters), polyphosphazenes, pseudo poly(amino acids), poly(glycerol-sebacate), copolymers thereof, and mixtures thereof. In a preferred embodiment, the resorbable synthetic polymers are selected from poly(lactic acids), poly(glycolic acids), poly(lactic-co-glycolic acids), poly(caprolactones) (PCLs), and mixtures thereof. Other curable bioresorbable elastomers include PCL derivatives, amino alcohol-based poly(ester amides) (PEA) and poly(octane-diol citrate) (POC). PCL-based polymers may require additional cross-linking agents such as lysine diisocyanate or 2,2-bis(-caprolacton-4-yl)propane to obtain elastomeric properties.

### Resorbable and Biodegradable Devices

There are applications of the disclosure that benefit from resorbable and biodegradable devices. For the purposes of the current disclosure, "resorbable" is intended to mean a device that breaks down and becomes assimilated *in vivo* (e.g., resorbable sutures) while "biodegradable" is intended to mean a device that is capable of being decomposed by bacteria or other living organisms post use. Nonlimiting, exemplary embodiments of both device types are given below.

*Resorbable Devices*

The main advantage of resorbable devices is that, in certain cases, they do not need to be removed once their drug cargo has been delivered. The resorbable implants described herein consist, at least in part, of materials that become degraded *in vivo* during the period of use. In some embodiments, the entire device is resorbable over the period of use. In some embodiments, *in vivo* degradation of the device occurs primarily after most of all the drug cargo has been released. In certain embodiments, one or more of the device components described above comprises a resorbable elastomer.

*Biodegradable Devices*

The impetus for biodegradable implants predominantly arises from the desire to minimize the detrimental environmental impact post use, i.e., waste minimization. For example, IVRs delivering the antiretroviral drug dapivirine are currently being evaluated for HIV prevention in large-scale clinical trials. These 28-day devices are made almost exclusively of silicone, which could result in a considerable waste burden if millions of women in sub-Saharan Africa regularly use the product, once approved. Over one million women around the world use the contraceptive IVR, NuvaRing®, which is predominantly made of EVA, another non-biodegradable elastomer creating further disposal concerns.

Compared to bioresorbable implants designed to degrade in the body to avoid the need for removal at the end of the period of use, biodegradable implants are designed to maintain integrity while inserted in the body, and to begin the degradation process once removed (i.e., post-use). One approach, similar to that used in biodegradable, disposable plastic items such as shopping bags and food containers, uses poly(lactic acid) polymers that are degraded by carboxyesterase enzymes produced by bacteria. An alternative approach is to utilize polymers that degrade in the presence of ultraviolet (UV) irradiation (i.e., sunlight). An important consideration is that the degradation process (and kinetics of degradation) be separated temporally from the period of use so that the delivery of the drug is not impacted by the degradation process during the implant period of use.

**Other Design Considerations**

*Considerations for the Delivery of Biomolecules*

Due to their large molecular weight, hydrophilicity, and chemical/physical instability, biomolecules (e.g., peptides, proteins, (ribo)nucleic acid oligomers) can be challenging to deliver in a controlled fashion from long-acting drug delivery devices. Many embodiments of the current disclosure overcome these limiting obstacles by immobilizing the biomolecules in porous kernels or water-soluble scaffolds (e.g., PVA nanofibers) encased in rate-limiting skins.

In addition to biomolecules that are approved by regulatory agencies to prevent or treat disease, the disclosure also serves as a platform to deliver exploratory agents for new applications. For example, in one non-limiting embodiment, messenger ribonucleic acids (mRNA's) -synthetic or natural- are delivered to stimulate the *in vivo* expression of one or more proteins, such as antibodies, and vaccine adjuvants. This approach has the advantage of leveraging the host's biochemical capabilities by stimulating it to synthesize the target agent *in vivo,* rather than delivering it directly from the implant. This can overcome high manufacturing costs of some biomolecules and their instability (e.g., cold-chain avoidance).

In some embodiments, certain excipients can improve the control of the biomolecule release rate from the implant (see "API Formulation"). For example, silk fibrin can be used to modulate the release rate of proteins.

In other embodiments, certain excipients can stabilize the biomolecules with respect to degradation or loss of biological activity using approaches known to those skilled in the art. Certain excipients stabilize biomolecules by creating a "water-like" environment in the dry state through hydrogen bonding interactions, e.g., sugars and amino acids. Other excipients create a glassy matrix that provides hydrogen bonding and immobilized the biomolecules to prevent aggregation that leads to loss of biologic activity (e.g., trehalose, inulin). Still other excipients can stabilize the pH in the implant formulation (e.g. buffer salts). Finally, surfactants can reduce the concentration of the biomolecules at the air-water interface during drying processes of formulation, decreasing shear stress and insoluble aggregate formation, and allowing the previously described stabilization mechanisms to occur throughout the drying process.

In Vivo *Localization of the Implant*

In various embodiments, one or more radio-opaque materials (e.g., barium sulfate) are incorporated into the elastomer implant shell (i.e., drug-impermeable polymer), or by making it into end plug to be used to seal the shell. The radio-opaque material can be integrated in the form of one or more band, or other shape, or dispersed throughout drug-impermeable polymer. In various embodiments, the elastomer material making up part of the implant is coated with a metal (e.g., titanium) to make it radio-opaque, using processes that are known in the art.

In certain embodiments, ultrasound is used to locate the implant. In these embodiments, polymers or polymer-additives (e.g., calcium) known in the art to be opaque to ultrasonography are employed to assist in visualizing the device *in vivo.*

The device may include at least one magnetic element to facilitate removal of the device (e.g., after drug delivery has been completed). In certain embodiments, the magnetic element may be located at the first end, the second end, or both the first and second ends of the cylindrical device. A soft polymeric coating may be provided over the magnetic elements.

To aid in implant insertion and/or removal, a hole may be punched, molded, or otherwise formed in one end of the implant. The hole may be used to grip the implant with forceps or another suitable tool. A loop made from suture material, wire, or other suitable material may be tied or otherwise attached to the hole to aid in gripping the implant for insertion and/or removal.

*Foreign Body Response*

Silicone implants, for example, are inexpensive and wieldy, but may elicit a foreign-body reaction and are prone to migration. ePTFE implants are more biocompatible and capable of ingrowth, but expensive. Silicone-ePTFE composites have a silicone core and ePTFE liner and are used in surgical applications, such as rhinoplasty and cheek-lip groove rejuvenation. In one embodiment, the elastomer implant sheath is bonded to an outer ePTFE sleeve to form a composite (i.e., the ePTFE sleeve only serves to mitigate the foreign body response and does not control or affect drug release from the device). In other embodiments, the ePTFE skin does play a role in controlling the API release rate from the device.

It is known in the art that the host's foreign body response can affect the safety of an implanted device, particularly for subcutaneous implants, or other types of devices implanted into body compartment. This reaction consists of protein adsorption on the implant surface, inflammatory cell infiltration, macrophage fusion into foreign body giant cells, fibroblast activation and ultimately fibrous encapsulation. This series of events may affect the function of subcutaneous implants, such as inhibition of drug diffusion from long-acting drug delivery depots and medical device failure. To date, combination approaches, such as hydrophilic coatings that reduce protein adsorption combined with delivery of dexamethasone are the most effective.

In a particular embodiment, the implantable drug delivery device releases one or more agents to mitigate or reduce the foreign body response in addition to the primary API. These agents are mixed with the API and any excipients, and formulated into the drug kernel (see "Drug Formulation", below). The agents are released from the implant with the API. In one embodiment, the agent included to reduce the foreign body response is a steroid. In one embodiment, this steroid is dexamethasone, or a dexamethasone derivative such as dexamethasone 21-acetate or dexamethasone 21-phosphate disodium salt.

It is known in the art that hydrogels, particularly zwitterionic hydrogels, can significantly reduce the foreign body response to subdermal implants.

*Fabrication of Porous Implant Components*

Porous material or materials can be used in implant fabrication either for the kernel or the skin, as described in detail above. In one embodiment, the API permeable portion of an implant device is formed from a porous membrane of polyurethane, silicone, or other suitable elastomeric material. Open cell foams and their production are known to those skilled in the art. Open cell foams may be produced using blowing agents, typically carbon dioxide or hydrogen gas, or a low-boiling liquid, present during the manufacturing process to form closed pores in the polymer, followed by a cell-opening step to break the seal between cells and form an interconnected porous structure through which diffusion may occur. An alternative embodiment employs a breath figure method to create an ordered porous polymer membrane for API release. In this method, a hexagonal array of micrometric pores is obtained by water droplet condensation during fast solvent evaporation performed under a humid flow. Porous membranes may also be fabricated using porogen leaching methods, whereby a polymer is mixed with salt or other soluble particles of controlled size prior to casting, spin-coating, extrusion, or other processing into a desired shape. The polymer composite is then immersed in an appropriate solvent, as known in the art, and the porogen particles are leached out leaving structure with porosity controlled by the number and size of leached porogen particles. A preferred approach is to use water-soluble particles and water as the solvent for porogen leaching and removal. Highly porous scaffolds with porosity values up to 93% and average pore diameters up to 500 $\mu$m can be formed using this technique. A variant of this method is melt molding and involves filling a mold with polymer powder and a porogen and heating the mold above the glass-transition temperature of the polymer to form a scaffold. Following removal from the mold, the porogen is leached out to form a porous structure with independent control of morphology (from porogen) and shape (from mold).

A phase separation process can also be used to form porous membranes. A second solvent is added to a polymer solution (quenching) and the mixture undergoes a phase separation to form a polymer-rich phase and a polymer-poor phase. The polymer-rich phase solidifies and the polymer poor phase is removed, leaving a highly porous polymer network, with the micro- and macro-structure controlled by parameters such as polymer concentration, temperature, and quenching rate. A similar approach is freeze drying, whereby a polymer solution is cooled to a frozen state, with solvent forming ice crystals and polymer aggregating in interstitial spaces. The solvent is removed by sublimation, resulting in an interconnected porous polymer structure. A final method for forming porous polymer membranes is using a stretching process to create an open-cell network as is known in the art.

Traditional implant designs well-known in the art involve the dissolution of the API(s) in the elastomer, the so-called "matrix design". In some exemplary embodiments disclosed in the art -for example the contraceptive IVR NuvaRing®- the matrix is surrounded by a thermoplastic polymer skin. Other traditional implant designs well-known in the art involve a solid API core surrounded by a continuous elastomer sheath, the so-called "reservoir design". In some exemplary embodiments disclosed in the art, the elastomer sheath consists of polyurethane and the API is contained as a powder or microtablets.

Non-traditional implant designs generally involve API tablets inserted into an elastomer scaffold, an approach used in drug delivery from IVRs. In some exemplary embodiments disclosed in the art, the tablet is uncoated with a polymer skin and drug release occurs through one or more channels fashioned in the elastomer support, which is impermeable to the API. In yet other exemplary embodiments disclosed in the art, the tablet is coated with a polymer skin and drug release occurs through one or more channels fashioned in the elastomer support, which is impermeable to the API.

Other examples of non-traditional implant designs include complex, open geometries produced by additive manufacturing. These designs essentially are a version of matrix-type devices and are made up of interconnected high surface area strands of API-polymer dispersions.

The disclosure is nonobvious. The key features are described in detail above and under "The Implantable Drug Delivery Device". Some exemplary, nonlimiting, nonobvious innovations include:

ePTFE as a rate-limiting, release-controlling skin that comprises microscopic pores that are a property of the ePTFE material and not created in a separate chemical (etching) or mechanical (punching) process step,

Controlled formation of open-cell, drug containing sponge scaffolds as kernels for sustained release drug delivery,

The combination of porogens and drug particles of defined size and size distribution in the controlled formation of open-cell, drug containing sponge scaffolds is novel and leads to drug release kinetics that are not predictable by one knowledgeable in the art,

Reservoir kernel made up of drug carriers with microstructure such as structured or layered particles and nanoparticles, or sponges,

Reservoir kernels with microstructure provided by fibers (random and oriented fibers as well as bundles, yarns, woven and non-woven mats composed of fibers). The fiber architecture provides a defined microstructure to the kernel that can be used to modulate release of drug from the implant and/or stabilize drug molecules in the kernel to degradation prior to release. The fiber-based kernel is surrounded by a skin that adds control of drug release kinetics,

Specific design considerations for the delivery of biomolecules, including mRNAs, antibodies and other proteins, nucleic acids (DNA, RNA), and peptide molecules, and/or

The hierarchical structure of implant device composition consisting of primary, secondary, and tertiary structural elements as described previously (see "The Implantable Drug Delivery Device").

The novel approaches to long-acting drug delivery described herein also are based on surprising laboratory results. The ability of the highly water-soluble compound TAF to diffuse through the highly hydrophobic ePTFE was unexpected. Equally unexpected was the observed linear TAF release rate and the degree of control over the drug release kinetics simply by changing the ePTFE density.

*Effect of Excipients on API Release*

The devices disclosed herein can comprise excipients to facilitate and/or control the release of the API from the devices. Nonlimiting examples of these excipients include PEG and TEC. It is contemplated that release kinetics of APIs can be modulated by the incorporation of different excipients into the devices disclosed herein. That is, the release kinetics of the API can be tuned over a wide range by changing the nature and/or amount of the excipient contained therein. In some cases, the devices contain low concentrations of excipient, e.g., from about 0% to about 30% excipient by weight. In some cases, the excipient is a polyether or an ester. In some cases, the excipient is PEG or TEC. In some cases, the devices comprise PEG to achieve a lower, sustained release of an API. In some cases, the devices comprise TEC to achieve a more immediate, larger dose of an API.

The disclosure also provides methods of delivering an API to subject via a device of the disclosure comprising a core comprising an excipient and an API (such as TAF) which is implanted in the subject. In some cases, the API is delivered with a consistent, sustained release profile. In some cases, the excipient is PEG or TEC.

**EXAMPLES**

*EXAMPLE 1 - Illustrative Buccal Implant Specifications for Tenofovir Alafenamide and HIV Prevention*

The disclosed buccal implant technology for the sustained, controlled delivery of APIs to systemic circulation is a platform technology and not directed at any specific API or application. The restrictions on the choice of API and, hence application, have been outlined under "Choice of API". An illustrative, non-restrictive example of the interplay between API physical, chemical, and biological properties and buccal implant characteristics is provided here.

Tenofovir alafenamide (TAF) is a nucleoside reverse transcriptase inhibitor (NRTI) and a potent antiretroviral drug against HIV. Preclinical and clinical studies suggest that TAF delivered systemically could safely prevent HIV infection in uninfected individuals. It is believed by many in the art that steady-state concentrations of tenofovir diphosphate (TFV-DP), the active metabolite of TAF, in peripheral blood mononuclear cells (PBMCs) are predictive of efficacy in preventing sexual HIV transmission. It is further believed by many in the art that TFV-DP concentrations in PBMCs of 50 fmol per million cells is a good target concentration for effective HIV prevention. A simulation using physiologically based PK (PB-PK) modeling estimated that a linear, subcutaneous release of TAF at a rate of 0.5 mg d$^{-1}$ would lead to the above protective TFV-DP PBMC concentrations (33). Another study estimated that lower TAF release rates of ca. 0.3 mg d$^{-1}$ could be protective (34). Assuming that buccal TAF delivery is equally efficient as subcutaneous delivery, two buccal implants of the design **206,** shown in **FIG 10** (dimensions, 2.5 mm dia., 40 mm, length; volume, 196 mm$^3$, TAF content, 75% w/v) each containing 130 mg TAF and delivering at 0.25 mg d$^{-1}$ each, with zero order kinetics, could prevent HIV infection for up to one year.

*EXAMPLE 2 - Illustrative Buccal Implant Specifications for Cabotegravir and HIV Prevention*

The disclosed buccal implant technology for the sustained, controlled delivery of APIs to systemic circulation is a platform technology and not directed at any specific API or application. The restrictions on the choice of API and, hence application, have been outlined under "Choice of API". An illustrative, non-restrictive example of the interplay between API physical, chemical, and biological properties and buccal implant characteristics is provided here.

Cabotegravir (CAB) is a potent strand-transfer integrase inhibitor being developed for HIV treatment and prevention. It is believed by many in the art that steady-state plasma concentrations of CAB are predictive of efficacy in preventing sexual HIV transmission. It is further believed by many in the art that steady state plasma CAB concentrations of 0.66 $\mu$g mL$^{-1}$, or four times the protein adjusted IC$_{90}$ (PA-IC$_{90}$) concentration from non-human primate efficacy studies (*35, 36*), are a good target for effective HIV prevention. The target was adjusted subsequently based on human clinical PK data (*37*). A phase 2a trial evaluating an injectable, intramuscular, long-acting CAB formulation suggests that male and female participants dosed with 600 mg every 8 weeks met the targets of 80% and 95% of participants with trough concentrations above 4$\times$ and 1$\times$PA-IC$_{90}$, respectively (38). Due to the tailing (i.e., non-steady state) PK profile of the injectable CAB formulation (*37, 38*), a lower dose or longer duration should be achievable from a buccal CAB implant with linear *in vivo* drug release profiles. It is estimated that two buccal implants of the geometry **108,** shown in **FIG 4,** of design **206,** shown in **FIG 10,** (dimensions, 5 mm $\times$ 2.5 mm $\times$ 35 mm; volume, 463 mm$^2$, CAB content, 65% w/v) each containing 250 mg CAB and delivering at 2.2 mg d$^{-1}$ each, with zero order kinetics, could prevent HIV infection for up to three months.

*EXAMPLE 3 - Illustrative Buccal Implant Specification Calculator*

A nonlimiting example of an algorithm for calculating the buccal implant specifications for any application is given below.

$$V = \frac{(RR \times t)}{SF \times m_f \times \rho}$$

where,

$V$ (mL) is the total implant volume (i.e., volume of single implant or sum of volumes of multiple implants),
$RR$ (g d$^{-1}$) is the total drug release rate of the implant(s). For nonlinear release rates, $RR$ corresponds to the integral of cumulative drug release (*y*-axis) over time (*x*-axis) for the period of use, divided by $t$,
$t$ (d) is the duration of use,
$SF$ is a dimensionless scaling factor, typically between 0.50 and 0.99 to ensure that sufficient drug remains in the implant to maintain the target drug delivery profile over the period of use,
$m_f$ is the mass fraction of drug in the implant(s), typically between 0.25 and 0.95, to account for the presence of excipients,
$\rho$ (g mL$^{-1}$) is the density of the implant(s)

The value of $RR$ will be determined in part by the potency of the drug and how efficiently it distributes to the target compartment(s) to achieve consistent pharmacologic efficacy. In many cases $RR$ will need to be determined in preclinical studies and confirmed clinically.

*EXAMPLE 4 - Sustained Release of Tenofovir Alafenamide from ePTFE Tubes*

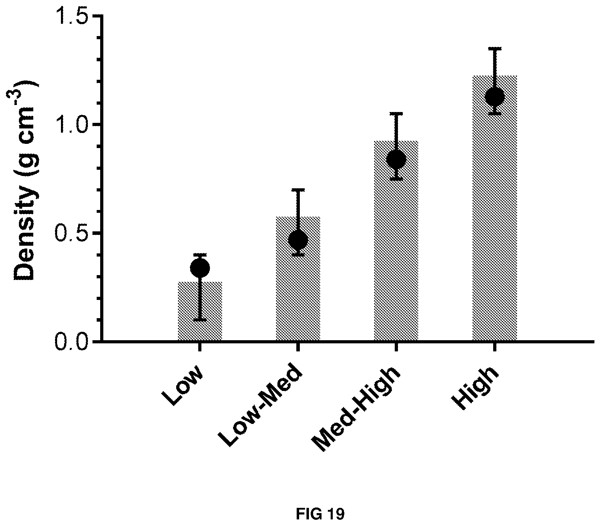

Custom-manufactured ePTFE tubes (outer diameter, 2.4 mm; wall thickness, 0.2 mm), manufactured through a precision extrusion process (Aeos™ technology), were supplied by Zeus Industrial Products, Inc. (Orangeburg, SC). The four ePTFE tubing densities (see **FIG. 15**) were designed to span the range of practical values for biomedical manufacturing (i.e., medical-grade). Target ePTFE densities are shown in **FIG. 19.** The physical data for the extrudates are provided in **TABLE 1** below.

**TABLE 1.** Custom ePTFE Tubing Specifications (target and measured).

| # | Target Specifications | | | Actual Specifications | | |
|---|---|---|---|---|---|---|
| | Density (g cm$^{-3}$) | Inner Dia. (mm) | Wall (mm) | Density (g cm$^{-3}$) | Inner Dia. (mm) | Wall (mm) |
| 1 | $0.25 \pm 0.15$ | $2.01 \pm 0.13$ | 0.127 +0.13/- 0.038 | 0.34 | 2.057 | 0.178 |
| 2 | $0.55 \pm 0.15$ | $2.01 \pm 0.13$ | 0.127 +0.13/- 0.038 | 0.47 | 1.984 | 0.178 |
| 3 | $0.9 \pm 0.15$ | $2.01 \pm 0.13$ | 0.127 +0.13/- 0.051 | 0.84 | 1.981 | 0.203 |
| 4 | $1.2 \pm 0.15$ | $2.01 \pm 0.13$ | 0.127 +0.13/- 0.051 | 1.13 | 1.996 | 0.203 |

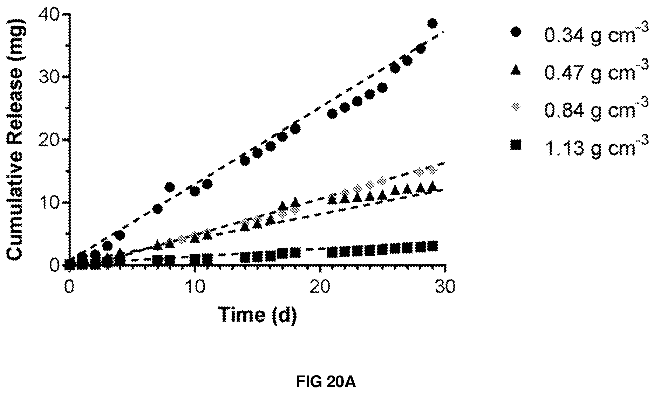

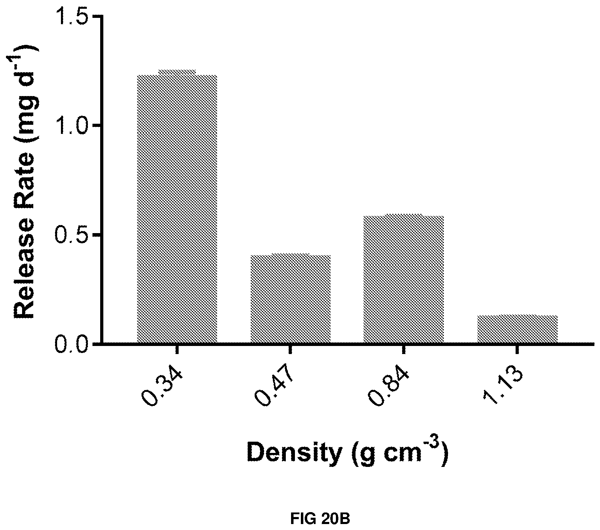

Segments (25 mm long) of the ePTFE tubes listed in **TABLE 1** were cut, and approximately 3 mm of each end of the tube were prepared for sealing using FluoroEtch primer (Action Technologies, Pittston, PA) according to manufactures instructions. The tubes were then filled with tenofovir alafenamide (TAF) powder. No other excipients were used. The mass of TAF per implant varied between 20 and 70 mg depending on the experiment. The filled implants were sealed at both ends by placing a drop of Permabond 105 cyanoacrylate adhesive (Permabond Engineering Adhesives, Pottstown, PA, USA) inside each end of the tube and crimping the end closed for 30 sec to allow the adhesive to cure and form a tight seal. Implants were kept at room temperature (ca. 23°C) for 24 hours to allow the adhesive bond to reach full strength. The TAF *in vitro* release kinetics from the loaded implants was investigated by immersing the device in a solution consisting of phosphate buffered saline (PBS, $1\times$, 100 mL) containing sodium azide (0.01% w/v) at 37°C with orbital shaking at 125 RPM for 30 days. The concentration of TAF in the release media was measured by UV-vis absorption spectroscopy ($\lambda_{max}$ 262 nm). The release rates are shown on **FIG. 16.** Release rates for the ePTFE tubes of **TABLE 1** are given in **FIG. 20A,** with rates as a function of ePTFE density given in **FIG. 20B.**

*EXAMPLE 5 - Effect of TEC on TAF Release rates*

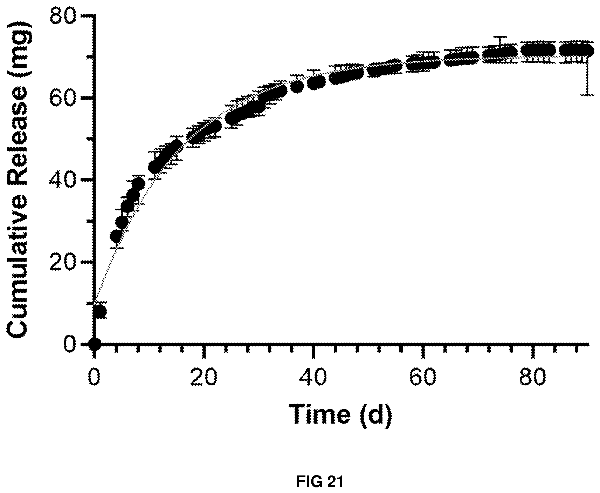

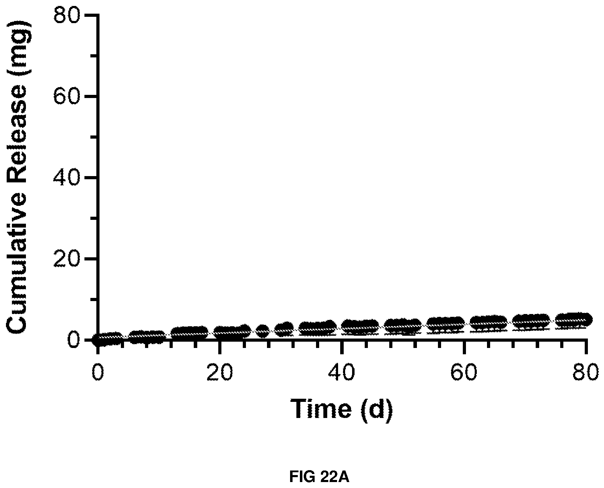

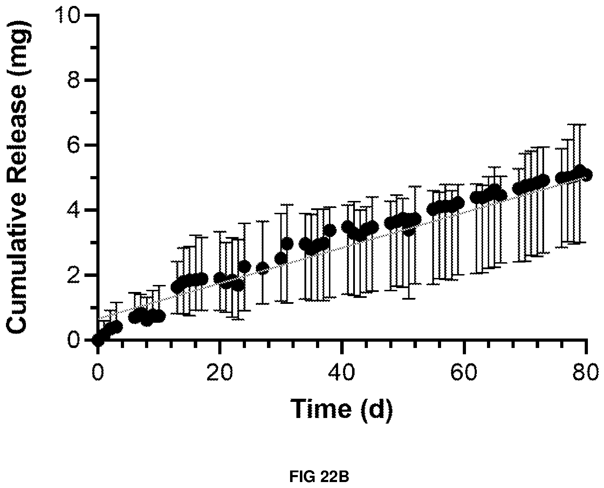

Both triethyl citrate (TEC) and PEG 400 are liquid excipients commonly used in the art. The implants used in the *in vitro* studies shown in **FIG. 21, 22A,** and **22B** only differ by the formulation of the core. In both cases, the cores consist of TAF blended into a paste with TEC (70% w/w TAF, **FIG. 21**) or PEG 400 (73% w/w TAF, **FIG. 22A** and **FIG. 22B**).

The 90-day cumulative TAF release (median $\pm$ 95% *CI*) from 40 mm long, 2.4 mm outer dia. ePTFE ($\rho = 0.84$ g cm$^{-3}$) implants ($N = 6$) filled with a paste ($141.8 \pm 2.3$ mg) consisting of TAF (70% w/w) blended with TEC (30% w/w) is shown in **FIG. 21.** The TAF *in vitro* release kinetics from the loaded implants was investigated by immersing the device in a solution consisting of phosphate buffered saline (PBS, $1\times$, 100 mL) containing sodium azide (0.01% w/v) and solutol (0.5% w/v) at 37°C with orbital shaking at 125 RPM for 30 days. The concentration of TAF in the release media was measured by UV-vis absorption spectroscopy ($\lambda_{max}$ 262 nm). The data were analyzed using an exponential, one-phase decay least squares fit model (grey line) to afford a measured release half-life of 11.1 d ($R^2 = 0.9660$).

The 80-day cumulative TAF release (median $\pm$ 95% *CI*) from 40 mm long, 2.4 mm outer dia. ePTFE ($\rho = 0.84$ g cm$^{-3}$) implants ($N = 4$) filled with a paste ($140.8 \pm 2.2$ mg) consisting of TAF (77% w/w) blended with PEG 400 (23% w/w) is shown in **FIG. 22A** and **FIG. 22B.** The TAF *in vitro* release kinetics from the loaded implants was investigated by immersing the device in a solution consisting of phosphate buffered saline (PBS, $1\times$, 100 mL) containing sodium azide (0.01% w/v) and solutol (0.5% w/v) at 37°C with orbital shaking at 125 RPM for 30 days. The concentration of TAF in the release media was measured by UV-vis absorption spectroscopy ($\lambda_{max}$ 262 nm). The data were analyzed using a simple linear regression fit model (grey line) to afford a measured slope (release rate) of 54.4 $\mu$g d$^{-1}$ ($R^2 = 0.6254$).

Given the high TAF content in both implant groups, it was surprising that they exhibited drastically different TAF release profiles. When blended with TEC, 60 mg of TAF was delivered from the implants over the first month, with 5 mg delivered in less than the first day. On the other hand, PEG 400 dramatically reduced the TAF release rate, with ca. 5 mg delivered linearly over 80 days.

**REFERENCES CITED**

1. Coukell, A. J. et al. Drugs 1998, 55 (6), 861-887.
2. Le, J. et al., Ann. Pharmacother. 2001, 35 (3), 329-336.
3. Fischer, M. A., J. Obstet. Gynecol. Neonatal Nurs. 2008, 37 (3), 361-368.
4. Aagaard, K.; et al., Faseb J. 2013, 23 (3), 1012-1022.
5. Kuo, S.-H. et al. U.S. Patent 7,842,303 B2, Nov. 30, 2010.
6. Manavitehrani, I. et al, Polymers 2016, 8 (1).
7. Teo, A. J. T.; et al, ACS Biomater. Sci. Eng. 2016, 2 (4), 454-472.
8. Kuzma, P. et al. U.S. Patent 7,858,110 B2, Dec. 28, 2010.
9. Cima, M. J.; et al. U.S. Patent 9,586,035 B2, Dec. 11, 2008.
10. Conrad, K. et al, Arch. Facial Plast. Surg. 2008, 10 (4), 224-231.
11. Zelken, J. A. et al, Ann. Plast. Surg. 2017, 78 (2), 131-137.
12. Conrad, K. et al, J. Otolaryngol. 1992, 21 (3), 218-222.
13. Kastellorizios, M. et al, In Immune Responses to Biosurfaces: Mechanisms and Therapeutic Interventions, Lambris, J. D.; Ekdahl, K. N.; Ricklin, D.; Nilsson, B., Eds. 2015; Vol. 865, pp 93-108.
14. Vegas, A. J. et al, Nat. Biotechnol. 2016, 34 (3), 345-352.
15. DiCesare, P. et al. U.S. Pat. Appl. US 2016/0213904 A1, Aug. 5, 2010.
16. Jonathan, G.; et al, Int. J. Pharm. 2016, 499 (1-2), 376-394.
17. Liaw, C. Y.; et al, Biofabrication 2017, 9 (2).
18. Dickens Jr., E. D. et al. U.S. Patent 5,648,450, Jul. 15, 1997.
19. Kraibuhler, H. et al. U.S. Patent 9,889,604, Feb. 13, 2018.
20. Vidin, E.; et al, Contraception 2007, 76 (1), 35-39.
21. Persaud, T.; et al, Eur. Radiol. 2008, 18 (11), 2582-2585.
22. Mascarenhas, L., Contraception 1998, 58 (6), 79S-83S.
23. Nguyen, T.-T.; et al, In A Practical Guide to Office Gynecologic Procedures, 2nd ed. ed.; Blumenthal, P. D.; Berek, J. S., Eds. Lippincott Williams & Wilkins: Philadelphia, PA, 2013; pp 145-154.
24. de Las Vecillas Sánchez, L. et al, Int. J. Mol. Sci. 2017, 18 (6), E1316.
25. Wright, J. C.; et al, I In Long Acting Injections and Implants, Wright, J. C.; Burgess, D. J., Eds. 2012; pp 153-166.
26. Dunn, R. L.; et al. U.S. Patent 4,938,763A, Ju. 3, 1990.
27. Garrett, S.; et al, J. Dent. Res. 1996, 75, 1130-1130.
28. Herrlich, S. et al, Adv. Drug Deliv. Rev. 2012, 64 (14), 1617-1627.
29. Calo, E. et al, Eur. Polym. J. 2015, 65, 252-267.
30. FAQs Regarding Safe Practices for Medical Injections. https://www.cdc.gov/injectionsafety/PDF/FAQs-Safe-Practices-for-Medical-Injections.pdf (accessed Jun. 9).
31. WHO Best Practices for Injections and Related Procedures Toolkit; World Health Organization: Geneva, Switzerland, 2010; p 69.
32. Phillips, K. T. et al, J. Subst. Abus. Treat. 2012, 43 (3), 313-321.
33. Kumar, R.; et al. In In Silico Simulation of Long-acting Tenofovir Alafenamide Subcutaneous Implant, 2019 Conference on Retroviruses and Opportunistic Infections (CROI), Seattle, WA, Mar. 4-7, 2019; CROI, Alexandria, VA: Seattle, WA, 2019; p Abstract Number 487.
34. Gunawardana, M. et al, Antimicrob. Agents Chemother. 2015, 59 (7), 3913-3919.
35. Andrews, C. D.; et al, Science 2014, 343 (6175), 1151-1154.
36. Andrews, C. D.; et al, Sci. Transl. Med. 2015, 7 (270).
37. Markowitz, M.; et al, Lancet HIV 2017, 4 (8), E331-E340.
38. Landovitz, R. J. et al, PLoS Med. 2018, 15 (11).

**Claims**

1. A buccal implant device configured to provide sustained drug delivery, the buccal implant device comprising:

   a body having a non-cylindrical geometry, the body adapted to be disposed within the oral mucosa of a patient; and
   one or more active pharmaceutical ingredients disposed within the body.

2. The buccal implant device of claim 1, further comprising a hole formed in the body, optionally further comprising a loop coupled to the hole to guide the body into or out of the oral cavity.

3. A buccal implant device configured to provide sustained drug delivery, the buccal implant device comprising:

   a body adapted to be disposed within the oral mucosa of a patient;
   means for guiding the body into or out of the oral cavity of a patient; and
   one or more active pharmaceutical ingredients disposed within the body,
   optionally wherein the body has a non-cylindrical geometry.

4. The buccal implant device of claim 3, wherein the means for guiding the body into or out of the oral cavity comprises a hole formed in the body, optionally wherein the means for guiding the body into or out of the oral cavity further comprises a loop coupled to the hole.

5. The buccal implant device of claim 3 or 4, wherein the means for guiding the body into or out of the oral cavity comprises one or more tapers of the body and/or one or more curved edges of the body.

6. The buccal implant device of any one of claims 1 to 5, wherein the buccal implant device is of a matrix-type, a reservoir type, or a hybrid type.

7. The buccal implant device of any one of claims 1 to 6, wherein the body has a saw shape, a saber shape, or a ribbed shape.

8. The buccal implant device any one of claims 1 to 7, wherein the body is tapered, optionally wherein the body includes a single taper, two tapers, or three tapers.

9. The buccal implant device of any one of claims 1 to 8, wherein the body has one or more curved edges configured to direct the implant into a specific orientation within the oral cavity, optionally wherein the one or more curved edges form a point at an end of the body.

10. The buccal implant device of any one of claims 1 to 9, wherein the device has a shape or geometry which minimizes impediment of physical jaw function and/or discomfort to the patient.

11. The buccal implant device of any one of claims 1 to 10, wherein the device comprises expanded polytetrafluoroethylene (ePTFE) having microscopic pores, optionally wherein the ePTFE has a density of about 0.25 to about 1.5 g cm$^{-3}$.

12. The buccal implant device of any one of claims 1 to 11, wherein the body comprises a sealed tube, optionally wherein the sealed tube has an inner diameter of about 1 mm to about 3 mm and/or a wall thickness of about 0.1 to 0.3 mm, preferably wherein the sealed tube has an inner diameter of about 2 mm and/or a wall thickness of about 0.125 mm.

13. The buccal implant device of any one of claims 1 to 12, wherein the one or more active pharmaceutical ingredients are selected from antiretrovirals, antimicrobial agents, antibacterial agents, antivirals, hormones, statins, β-blockers, ACE inhibitors, angiotensin receptor blockers, vitamins, steroids, biologics, anti-cancer drugs, allergy medications, anticoagulants, antiplatelet therapies, non-steroidal anti-inflammatory drugs, vaccines, and combinations thereof, optionally wherein the one or more active pharmaceutical ingredients comprise atazanavir, didanosine, efavirenz, emtricitabine, lamivudine, lopinavir, nevirapine, raltegravir, ritonavir, saquinavir, stavudine, tenofovir, tenofovir disoproxil fumarate, tenofovir alafenamide, zidovudine, acyclovir, famciclovir, valcyclovir, morphine, buprenorphine, estrogen, progestin, progesterone, cyclosporine, a calcineurin inhibitor, prostaglandin, cabotegravir, rilpivirine, lenacapavir (GS-6207), 4'-ethynyl-2-fluoro-2'-deoxyadenosine (MK-8591, EFdA), a beta-blocker, gentamycin, corticosteroid, a fluoroquinolone, insulin, an antineoplastic drug, anti-nausea drug, a corticosteroid, an antibiotic, morphine buprenorphine, a VEGF inhibitor, or combinations thereof, preferably wherein the one or more active pharmaceutical ingredients comprise tenofovir.

14. The buccal implant device of any one of claims 1 to 13, wherein the device is configured to release the one or more active pharmaceutical ingredients over a period of 1 to 12 months.

15. The buccal implant device of claim 14, wherein the device is configured to release the one or more active pharmaceutical ingredients over a period of 3 months.

**Patentansprüche**

1. Wangenimplantatvorrichtung, die so konfiguriert ist, dass sie eine anhaltende Arzneimittelabgabe bereitstellt, wobei die Wangenimplantatvorrichtung Folgendes umfasst:

   einen Körper, der eine nicht-zylindrische Geometrie aufweist, wobei der Körper so angepasst ist, dass er innerhalb der Mundschleimhaut eines Patienten angebracht werden kann; und
   einen oder mehrere pharmazeutische Wirkstoffe, die innerhalb des Körpers angebracht sind.

2. Wangenimplantatvorrichtung nach Anspruch 1, ferner umfassend ein Loch, das in dem Körper ausgebildet ist, und optional eine Schlaufe, die mit dem Loch gekoppelt ist, um den Körper in die oder aus der Mundhöhle zu führen.

3. Wangenimplantatvorrichtung, die so konfiguriert ist, dass sie eine anhaltende Arzneimittelabgabe bereitstellt, wobei die Wangenimplantatvorrichtung Folgendes umfasst:
   einen Körper, der so angepasst ist, dass er innerhalb der Mundschleimhaut eines Patienten angebracht wird; Mittel zum Führen des Körpers in die oder aus der Mundhöhle eines Patienten; und einen oder mehrere pharmazeutische Wirkstoffe, die innerhalb des Körpers angebracht sind, wobei der Körper optional eine nicht-zylindrische Geometrie aufweist.

4. Wangenimplantatvorrichtung nach Anspruch 3, wobei das Mittel zum Führen des Körpers in die oder aus der Mundhöhle ein Loch umfasst, das in dem Körper ausgebildet ist, wobei das Mittel zum Führen des Körpers in die oder aus der Mundhöhle optional ferner eine Schlaufe umfasst, die mit dem Loch gekoppelt ist.

5. Wangenimplantatvorrichtung nach Anspruch 3 oder 4, wobei das Mittel zum Führen des Körpers in die oder aus der Mundhöhle eine oder mehrere Verjüngungen des Körpers und/oder eine oder mehrere gebogene Kanten des Körpers umfasst.

6. Wangenimplantatvorrichtung nach einem der Ansprüche 1 bis 5, wobei die Wangenimplantatvorrichtung vom Matrixtyp, vom Reservoirtyp oder vom Hybridtyp ist.

7. Wangenimplantatvorrichtung nach einem der Ansprüche 1 bis 6, wobei der Körper eine Sägeform, eine Säbelform oder eine gerippte Form aufweist.

8. Wangenimplantatvorrichtung nach einem der Ansprüche 1 bis 7, wobei der Körper verjüngt ist, wobei der Körper wahlweise eine einzelne Verjüngung, zwei Verjüngungen oder drei Verjüngungen beinhaltet.

9. Wangenimplantatvorrichtung nach einem der Ansprüche 1 bis 8, wobei der Körper eine oder mehrere gebogene Kanten aufweist, die so konfiguriert sind, dass sie das Implantat in eine spezifische Ausrichtung innerhalb der Mundhöhle lenken, wobei die eine oder mehrere gebogene Kanten optional eine Spitze an einem Ende des Körpers ausbilden.

10. Wangenimplantatvorrichtung nach einem der Ansprüche 1 bis 9, wobei die Vorrichtung eine Form oder Geometrie aufweist, die eine Beeinträchtigung der physischen Kieferfunktion und/oder Beschwerden für den Patienten minimiert.

11. Wangenimplantatvorrichtung nach einem der Ansprüche 1 bis 10, wobei die Vorrichtung expandiertes Polytetrafluorethylen (ePTFE) umfasst, das mikroskopische Poren aufweist, wobei das ePTFE optional eine Dichte von etwa 0,25 bis etwa 1,5 g/cm$^{-3}$ aufweist.

12. Wangenimplantatvorrichtung nach einem der Ansprüche 1 bis 11, wobei der Körper ein versiegeltes Rohr umfasst, wobei das versiegelte Rohr optional einen Innendurchmesser von etwa 1 mm bis etwa 3 mm und/oder eine Wandstärke von etwa 0,1 bis 0,3 mm aufweist, wobei das versiegelte Rohr vorzugsweise einen Innendurchmesser von etwa 2 mm und/oder eine Wandstärke von etwa 0,125 mm aufweist.

13. Wangenimplantatvorrichtung nach einem der Ansprüche 1 bis 12, wobei der eine oder mehrere pharmazeutische Wirkstoffe ausgewählt sind aus antiretroviralen Mitteln, antimikrobiellen Mitteln, antibakteriellen Mitteln, antiviralen Mitteln, Hormonen, Statinen, ß-Blockern, ACE-Hemmern, Angiotensinrezeptorblockern, Vitaminen, Steroiden, Biologika, Krebsmedikamenten, Allergiemedikamente, Antikoagulanzien, Thrombozytenaggregationshemmer, nicht-steroidale entzündungshemmende Medikamente, Impfstoffe und Kombinationen davon, wobei der eine oder mehrere pharmazeutische Wirkstoffe optional Atazanavir, Didanosin, Efavirenz, Emtricitabin, Lamivudin, Lopinavir, Nevirapin, Raltegravir, Ritonavir, Saquinavir, Stavudin, Tenofovir, Tenofovirdisoproxilfumarat, Tenofoviralafenamid, Zidovudin, Acyclovir, Famciclovir, Valcyclovir, Morphin, Buprenorphin, Östrogen, Progestin, Progesteron, Cyclosporin, ein Calcineurin-Inhibitor, Prostaglandin, Cabotegravir, Rilpivirin, Lenacapavir (GS-6207), 4'-Ethinyl-2-fluor-2'-desoxyadenosin (MK-8591, EFdA), ein Betablocker, Gentamycin, ein Kortikosteroid, ein Fluorchinolon, Insulin, ein antineoplastisches Medikament, ein Medikament gegen Übelkeit, ein Kortikosteroid, ein Antibiotikum, Morphin, Buprenorphin, ein VEGF-Inhibitor oder Kombinationen davon sind, wobei der eine oder mehrere pharmazeutische Wirkstoffe vorzugsweise Tenofovir umfassen.

14. Wangenimplantatvorrichtung nach einem der Ansprüche 1 bis 13, wobei die Vorrichtung so konfiguriert ist, dass sie den einen oder mehrere pharmazeutische Wirkstoffe über einen Zeitraum von 1 bis 12 Monaten freisetzt.

15. Wangenimplantatvorrichtung nach Anspruch 14, wobei die Vorrichtung so konfiguriert ist, dass sie den einen oder mehrere pharmazeutische Wirkstoffe über einen Zeitraum von 3 Monaten freisetzt.

**Revendications**

1. Dispositif d'implant buccal configuré pour l'administration prolongée de médicament, le dispositif d'implant buccal comprenant :

   un corps présentant une géométrie non cylindrique, le corps étant adapté pour être disposé à l'intérieur de la muqueuse buccale d'un patient ; et
   un ou plusieurs principes pharmaceutiquement actifs disposés à l'intérieur du corps.

2. Dispositif d'implant buccal selon la revendication 1, comprenant en outre un trou formé dans le corps, comprenant en outre en option une boucle couplée au trou pour guider le corps dans et hors de la cavité buccale.

3. Dispositif d'implant buccal configuré pour l'administration prolongée de médicament, le dispositif d'implant buccal comprenant :

   un corps adapté pour être disposé à l'intérieur de la muqueuse buccale d'un patient ;
   des moyens de guidage du corps dans ou hors de la cavité buccale d'un patient ; et
   un ou plusieurs principes pharmaceutiquement actifs disposés à l'intérieur du corps, en option dans lequel le corps présente une géométrie non cylindrique.

4. Dispositif d'implant buccal selon la revendication 3, dans lequel les moyens de guidage du corps dans ou hors de la cavité buccale comprennent un trou formé dans le corps, en option dans lequel les moyens de guidage du corps dans ou hors de la cavité buccale comprennent en outre une boucle couplée au trou.

5. Dispositif d'implant buccal selon la revendication 3 ou 4, dans lequel les moyens de guidage du corps dans ou hors de la cavité buccale comprennent un ou plusieurs rétrécissements du corps et/ou un ou plusieurs bords courbés du corps.

6. Dispositif d'implant buccal selon l'une quelconque des revendications 1 à 5, dans lequel le dispositif d'implant buccal est d'un type de matrice, d'un type de réservoir ou d'un type hybride.

7. Dispositif d'implant buccal selon l'une quelconque des revendications 1 à 6, dans lequel le corps présente une forme de scie, une forme de sabre ou une forme nervurée.

8. Dispositif d'implant buccal selon l'une quelconque des revendications 1 à 7, dans lequel le corps est rétréci, en option dans lequel le corps comporte un rétrécissement unique, deux rétrécissements ou trois rétrécissements.

9. Dispositif d'implant buccal selon l'une quelconque des revendications 1 à 8, dans lequel le corps présente un ou plusieurs bords courbés configurés pour diriger l'implant dans une orientation spécifique à l'intérieur de la cavité buccale, en option dans lequel les un ou plusieurs bords courbés forment un point sur une extrémité du corps.

10. Dispositif d'implant buccal selon l'une des revendications 1 à 9, dans lequel le dispositif présente une forme ou une géométrie qui minimise l'entrave d'une fonction physique de mâchoire et/ou la gêne pour le patient.

11. Dispositif d'implant buccal selon l'une quelconque des revendications 1 à 10, dans lequel le dispositif comprend du polytétrafluoroéthylène expansé (ePTFE) présentant des pores microscopiques, en option dans lequel l'ePTFE présente une densité d'environ 0,25 à environ 1,5 g/cm$^{-3}$.

12. Dispositif d'implant buccal selon l'une quelconque des revendications 1 à 11, dans lequel le corps comprend un tube étanchéifié, en option dans lequel le tube étanchéifié présente un diamètre intérieur d'environ 1 mm à environ 3 mm et/ou une épaisseur de paroi d'environ 0,1 à 0,3 mm, de préférence dans lequel le tube étanchéifié présente un diamètre intérieur d'environ 2 mm et/ou une épaisseur de paroi d'environ 0,125 mm.

13. Dispositif d'implant buccal selon l'une quelconque des revendications 1 à 12, dans lequel les un ou plusieurs principes pharmaceutiquement actifs sont choisis parmi des antirétroviraux, des agents antimicrobiens, des agents antibactériens, des antiviraux, des hormones, des statines, des ß-bloquants, des inhibiteurs ACE, des inhibiteurs de récepteurs d'angiotensine, des vitamines, des stéroïdes, des produits biologiques, des médicaments anticancer, des médicaments contre les allergies, des anticoagulants, des traitements antiplaquettaires, des médicaments anti-inflammatoires non stéroïdiens, des vaccins, et des combinaisons de ceux-ci, dans lequel en option les un ou plusieurs principes pharmaceutiquement actifs comprennent de l'Atazanavir, de la Didanosine, de l'éfavirenz, de l'emtricitabine, de la lamivudine, du lopinavor, de la névirapine, du raltégravir, du ritonavir, du saquinavir, de la Stavudine, du ténovir, du fumarate de ténovir disoproxil, du ténovir alafénamide, de la zidovudine, de l'acyclovir, du famciclovir, du valcyclovir, de la morphine, de la buprénorphine, des œstrogènes, de la progestine, de la progestérone, de la cyclosporine, un inhibiteur de calcineurine, de la prostaglandine, du cabotégravir, de la rilpivirine, du lénacapavir (GS-6207), de la 4'-éthnynyl-2-fluoro-2'-déoxyadénosine (MK-8591, EFdA), un bêtabloquant, de la gentamicine, des corticostéroïdes, de la fluoroquinolone, de l'insuline, un médicament antinéoplastique, un médicament antiémétique, un corticostéroïde, un antibiotique, de la buprénorphine, un anti-VEGF ou des combinaisons de ceux-ci, de préférence dans lequel les un ou plusieurs principes pharmaceutiquement actifs comprennent du ténofovir.

14. Dispositif d'implant buccal selon l'une quelconque des revendications 1 à 13, dans lequel le dispositif est configuré pour libérer les un ou plusieurs principes pharmaceutiquement actifs sur une période de 1 à 12 mois.

15. Dispositif d'implant buccal selon la revendication 14, dans lequel le dispositif est configuré pour libérer les un ou plusieurs principes pharmaceutiquement actifs sur une période de 3 mois.

**FIG 1A.**

**FIG 1B.**

**FIG 2**.

**FIG 3**.

108c
End view

108

Top view    Side view
108a         108b

**FIG 4.**

109

Top view

Side view

109a

End view

**FIG 5.**

Side view

Top view

Side view

End view

110

**FIG 6.**

111

112

**FIG 7**.

200

201                    202                    203

**FIG 8**.

204a

204b

204c

204

205a

205

FIG 9.

206

z
y
x

206a
206b

206a
206b

Cross-section
In y-z plane

Cross-section
In x-z plane

206a
206b

206a
206b

FIG 10.

FIG 11.

300

z

y

x

301

302

Cross-section
In y-z plane

301

302

Cross-section
In x-z plane

**FIG 12.**

303b

303b    303a

303a

303

304b

304a    304b    304c

304c    304a

304

**FIG 13**.

FIG 14.

Cross-section
In y-z plane

Cross-section
In x-z plane

**FIG 15.**

FIG 16.

415

Cross-sections
In x-z plane

419

416        417        418

420

FIG 17.

FIG 18.

FIG 19

FIG 20A

FIG 20B

FIG 21

FIG 22A

FIG 22B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62911765
- US 62941267
- US 63061483
- WO 2016149561 A1
- US 4020558 A
- US 7842303B2 A, Kuo, S.-H.

- US 7858110B2 A, Kuzma, P.
- US 9586035B2 A, Cima, M. J.
- US 20160213904 A1, DiCesare, P
- US 5648450 A, Dickens Jr., E. D.
- US 9889604 B, Kraibuhler, H
- US 4938763 A, Dunn, R. L

### Non-patent literature cited in the description

- **ALLEN et al.** Remington: The Science and Practice of Pharmacy. Pharmaceutical Press, 15 September 2012
- **HORNYAK et al.** Introduction to Nanoscience and Nanotechnology. CRC Press, 2008
- Oxford Textbook of Medicine. Oxford Univ. Press, May 2010
- Harrison's Principles of Internal Medicine. McGraw-Hill, 2018, vol. 1
- **SINGLETON** ; **SAINSBURY**. Dictionary of Microbiology and Molecular Biology. J. Wiley & Sons, 2006
- **SMITH**. March's Advanced Organic Chemistry Reactions, Mechanisms and Structure. J. Wiley & Sons, 2013
- **SINGLETON**. Dictionary of DNA and Genome Technology. Wiley-Blackwell, 2012
- **COUKELL, A. J. et al.** *Drugs*, 1998, vol. 55 (6), 861-887
- **LE, J. et al.** *Ann. Pharmacother.*, 2001, vol. 35 (3), 329-336
- **FISCHER, M. A.** *J. Obstet. Gynecol. Neonatal Nurs*, 2008, vol. 37 (3), 361-368
- **AAGAARD, K et al.** *Faseb J.*, 2013, vol. 23 (3), 1012-1022
- **MANAVITEHRANI, I. et al.** *Polymers*, 2016, vol. 8 (1)
- **TEO, A. J. T.; et al.** *ACS Biomater. Sci. Eng*, 2016, vol. 2 (4), 454-472
- **CONRAD, K. et al.** *Arch. Facial Plast. Surg*, 2008, vol. 10 (4), 224-231
- **ZELKEN, J. A. et al.** *Ann. Plast. Surg*, 2017, vol. 78 (2), 131-137
- **CONRAD, K. et al.** *J. Otolaryngol*, 1992, vol. 21 (3), 218-222
- **KASTELLORIZIOS, M. et al.** Immune Responses to Biosurfaces: Mechanisms and Therapeutic Interventions. 2015, vol. 865, 93-108

- **VEGAS, A. J et al.** *Nat. Biotechnol.*, 2016, vol. 34 (3), 345-352
- **JONATHAN, G. et al.** *Int. J. Pharm.*, 2016, vol. 499 (1-2), 376-394
- **LIAW, C. Y. et al.** *Biofabrication*, 2017, vol. 9 (2)
- **VIDIN, E et al.** *Contraception*, 2007, vol. 76 (1), 35-39
- **PERSAUD, T.; et al.** *Eur. Radiol*, 2008, vol. 18 (11), 2582-2585
- **MASCARENHAS, L.** *Contraception*, 1998, vol. 58 (6), 79S-83S
- **NGUYEN, T.-T et al.** In A Practical Guide to Office Gynecologic Procedures,. Lippincott Williams & Wilkins, 2013, 145-154
- **DE LAS VECILLAS SÁNCHEZ, L. et al.** *Int. J. Mol. Sci.*, 2017, vol. 18 (6), E1316
- **WRIGHT, J. C et al.** I In Long Acting Injections and Implants. 2012, 153-166
- **GARRETT, S.; et al.** *J. Dent. Res.*, 1996, vol. 75, 1130-1130
- **HERRLICH, S. et al.** *Adv. Drug Deliv. Rev*, 2012, vol. 64 (14), 1617-1627
- **CALO, E et al.** *Eur. Polym. J.*, 2015, vol. 65, 252-267
- WHO Best Practices for Injections and Related Procedures Toolkit;. World Health Organization, 2010, 69
- **PHILLIPS, K. T. et al.** *J. Subst. Abus. Treat*, 2012, vol. 43 (3), 313-321
- **KUMAR, R et al.** In In Silico Simulation of Long-acting Tenofovir Alafenamide Subcutaneous Implant. *2019 Conference on Retroviruses and Opportunistic Infections (CROI)*, 04 March 2019
- **GUNAWARDANA, M. et al.** *Antimicrob. Agents Chemother*, 2015, vol. 59 (7), 3913-3919
- **ANDREWS, C. D.; et al.** *Science*, 2014, vol. 343 (6175), 1151-1154

- **ANDREWS, C. D et al.** *Sci. Transl. Med.*, 2015, vol. 7, 270
- **MARKOWITZ, M et al.** *Lancet HIV*, 2017, vol. 4 (8), E331-E340
- **LANDOVITZ, R. J. et al.** *PLoS Med.*, 2018, vol. 15 (11)